# DEMANDE DE BREVET EUROPEEN

(11) **EP 0 740 937 A2**
(43) Date de publication de la demande: **06.11.1996**
(21) Numéro de dépôt: 96400846.0
(22) Date de dépôt: 19.04.1996
(51) Int. Cl.: A61K 31/38, A61K 31/455, A61K 31/19, A61K 31/535, A61K 31/11, A61K 31/195, A61K 31/045, A61K 31/165, A61K 31/495, A61K 31/215, C07C 65/26

(54) **Utilisation de rétinoides pour la fabrication d'une composition cosmétique ou pharmaceutique**

(30) Priorité: 03.05.1995 FR 9505279
(71) Demandeur: CENTRE INTERNATIONAL DE RECHERCHES DERMATOLOGIQUES GALDERMA (C.I.R.D. GALDERMA), F-06560 Valbonne (FR)
(72) Inventeur: Bernardon, Jean-Michel, 06650 Le Rouret (FR)
(74) Mandataire: Andral, Christophe André Louis

(57) **Abrégé**

La présente invention concerne une utilisation de composés de type rétinoïdes en tant qu'agents actifs dans une composition cosmétique ou pour la fabrication d'une composition pharmaceutique, ces compositions étant destinées au traitement de désordres et/ou d'affections liés à une surrégulation des récepteurs RARs et/ou à une hypervitaminose A.

## Description

La présente invention concerne une utilisation de composés de type rétinoïdes en tant qu'agents actifs dans une composition cosmétique ou pour la fabrication d'une composition pharmaceutique, ces compositions étant destinées au traitement de désordres et/ou d'affections liés à une surrégulation des récepteurs RARs et/ou à une hypervitaminose A.

On sait que l'acide rétinoïque et certains de ses analogues (appelés également rétinoïdes) sont capables d'induire la différenciation des cellules (F9) de tératocarcinome embryonnaire de souris. La sécrétion de l'activateur du plasminogène qui accompagne cette différenciation est un indice de la réponse biologique des cellules F9 aux rétinoïdes. On sait également que la capacité de ces rétinoïdes à induire l'activateur du plasminogène est corrélée directement avec l'affinité qu'ils ont pour les récepteurs RARs (retinoic acid receptors) endogènes aux cellules F9 (Skin pharmacol., 1990 ; 3 : pp.256-267).

Il est connu également que des désordres ou des affections dermatologiques, rhumatismales, respiratoires, cardiovasculaires, osseuses ou encore ophtalmologiques sont notamment liées à une surrégulation (surexpression ou suractivité) des récepteurs RARs et/ou à une hypervitaminose A (présence dans l'organisme d'une quantité anormale de vitamine A ou de ses métabolites). Ainsi, on comprend l'intérêt de trouver des composés qui viennent inhiber les effets biologiques d'une surrégulation des récepteurs RARs et/ou d'une hypervitaminose A.

De manière tout à fait surprenante et inattendue, la Demanderesse a trouvé que certains composés de type rétinoïdes n'induisent pas la différenciation de ces cellules F9, mais se lient cependant aux RARs, cette liaison étant du type antagoniste.

Cette découverte est à la base de la présente invention.

La présente invention a donc trait à une utilisation d'une quantité efficace d'au moins un composé de type rétinoïdes de formule (I) en tant qu'agent actif dans une composition cosmétique ou pour la fabrication d'une composition pharmaceutique, lesdites compositions étant destinées au traitement de désordres ou d'affections liés à une surrégulation des récepteurs RARs et/ou à une hypervitaminose A.

Ces composés présentent une formule générale (I) : 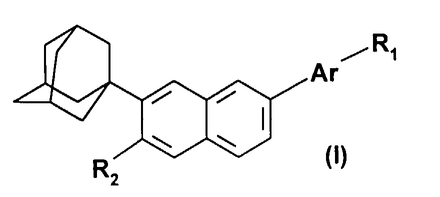 dans laquelle :
- R₁ représente
   (i) le radical -CH₃
   (ii) le radical -CH₂OH
   (iii) le radical -O-R₃
   (iv) le radical -CO-R₄
   R₃ et R₄ ayant les significations données ci-après,
- Ar est un radical de formule : 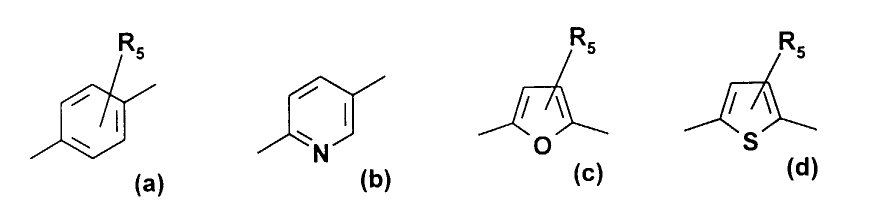 R₅ ayant la signification donnée ci-après,
- R₂ représente:
   (a) un radical -(X)ₙ-(CH₂)p-R₆
   (b) un radical -(X)ₙ -(CH₂)q-R₇
   (c) un radical -CH=CH-(CH₂)ₛ-R₆
   (d) un radical -CH=CH-(CH₂)ₜ-R₇
   R₆, R₇, X, n, p, q, s et t ayant les significations données ci-après, étant entendu que :
- R₃ représente un atome d'hydrogène, un radical alkyle inférieur, un radical -( CH₂)ₘ-(CO)ₙ -R₈.
   R₈, m et n ayant les significations données ci-après,
- R₄ représente:
   (a) un atome d'hydrogène,
   (b) un radical alkyle inférieur,
   (c) un radical de formule : 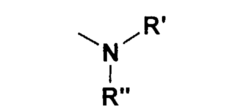
   (d) un radical -OR₉,
   R', R" et R₉ ayant les significations données ci-après,
- R₅ représente un atome d'hydrogène, d'halogène, un radical alkyle linéaire ou ramifié ayant de 1 à 20 atomes de carbone, un radical hydroxy, un radical -OR₁₀ ou -OCOR₁₀,
   R₁₀ ayant la signification donnée ci-après,
- R₆ représente un atome d'hydrogène, un radical alkyle linéaire ou ramifié ayant de 1 à 20 atomes de carbone, un radical alkényle, un radical alkynyle,
- R₇ représente un radical aryle, un radical mono ou polyhydroxyalkyle dont les hydroxy sont éventuellement protégés sous forme de méthoxy ou d'acétoxy ou d'acétonide, un radical amino-alkyle dont la fonction amine est éventuellement substituée par un ou deux groupements alkyles inférieurs, un radical polyéther, un radical -COR₄, un radical hétérocycle saturé ou insaturé ou encore un radical amino-aryle,
- R₈ représente un radical alkyle inférieur ou un hétérocycle saturé,
- R₉ représente un atome d'hydrogène, un radical alkyle linéaire ou ramifié, ayant de 1 à 20 atomes de carbone, un radical alkényle, un radical mono ou polyhydroxyalkyle, un radical aryle ou aralkyle éventuellement substitué(s) ou un reste de sucre ou un reste d'amino acide ou de peptide,
- R₁₀ représente un radical alkyle inférieur,
- X représente un atome d'oxygène ou un radical -S(O)ᵣ,
- R' et R", identiques ou différents, représentent un atome d'hydrogène, un radical alkyle inférieur, un radical mono- ou poly-hydroxyalkyle, un radical aryle éventuellement substitué ou un reste d'amino acide, de peptide ou de sucre ou encore pris ensemble forment un hétérocycle saturé,
- m est un nombre entier compris inclusivement entre 1 et 3,
- n est un nombre entier compris inclusivement entre 0 ou 1,
- p est un nombre entier compris inclusivement entre 5 et 12,
- q est un nombre entier compris inclusivement entre 0 et 12,
- r est un nombre entier compris inclusivement entre 0 et 2,
- s est un nombre entier compris inclusivement entre 3 et 10,
- t est un nombre entier compris inclusivement entre 0 et 10,
ainsi que leurs sels, et leurs analogues chiraux.

Ces composés de formule (I) peuvent donc également être des sels lorsque R₁ ou R₇ représente une fonction acide carboxylique et lorsque R₇ représente une fonction amine et les analogues chiraux et les isomères géométriques desdits composés de formule (I). Lorsque les composés selon l'invention se présentent sous forme de sels, il s'agit de préférence de sels d'un métal alcalin ou alcalinoterreux ou encore de zinc ou d'une amine organique.

Selon la présente invention, on entend par radical alkyle inférieur un radical ayant de 1 à 6 atomes de carbone, de préférence les radicaux méthyle, éthyle, isopropyle, butyle, tertiobutyle et hexyle.

Parmi les radicaux alkyle linéaire ou ramifié ayant de 1 à 20 atomes de carbone, on peut notamment citer les radicaux méthyle, éthyle, propyle, 2-éthyl-hexyle, octyle, dodécyle, hexadécyle et octadécyle.

Parmi les radicaux monohydroxyalkyle, on préfère un radical ayant 1 à 6 atomes de carbone, notamment un radical hydroxyméthyl, 2-hydroxyéthyle, 2-hydroxypropyle ou 3-hydroxypropyle.

Parmi les radicaux polyhydroxyalkyle, on préfère un radical contenant de 3 à 6 atomes de carbone et de 2 à 5 groupes hydroxyles, tels que les radicaux 2, 3-dihydroxypropyle, 2,3,4-trihydroxybutyle, 2,3,4,5-tétrahydroxypentyle ou le reste du pentaérythritol.

Parmi les radicaux aryle, on préfère un radical phényle éventuellement substitué par au moins un atome d'halogène, un hydroxyle ou une fonction nitro. Ainsi, parmi les radicaux amino-aryle, on préfère un radical amino-phényle éventuellement substitué par au moins un atome d'halogène, un hydroxyle ou une fonction nitro.

Parmi les radicaux aralkyle, on préfère le radical benzyle ou phénéthyle éventuellement substitué par au moins un atome d'halogène, un hydroxyle ou une fonction nitro.

Par radical alkényle, on entend un radical contenant de préférence de 2 à 5 atomes de carbone et présentant une ou plusieurs insaturations éthyléniques, tel que plus particulièrement le radical allyle.

Par reste de sucre, on entend un reste dérivant notamment de glucose, de galactose ou de mannose, ou bien encore de l'acide glucuronique.

Par reste d'aminoacide, on entend notamment un reste dérivant de la lysine, de la glycine ou de l'acide aspartique, et par reste de peptide on entend plus particulièrement un reste de dipeptide ou de tripeptide résultant de la combinaison d'acides aminés.

Par hétérocycle saturé, on entend de préférence un radical pipéridino, morpholino, pyrrolidino ou pipérazino, éventuellement substitué en position 4 par un radical alkyle en C₁-C₆ ou mono ou polyhydroxyalkyle tels que définis ci-dessus.

Par hétérocycle insaturé, on entend de préférence un radical pyridine, furanne ou thiophène.

Parmi les atomes d'halogène, on préfère un atome de fluor, de chlore ou de brome.

Par radical amino-alkyle, on entend un radical contenant de préférence de 1 à 6 atomes de carbone, notamment les radicaux amino-méthyle, 3-amino-propyle, 6-amino-hexyle.

Par radical polyéther, on entend un radical contenant de préférence de 1 à 6 atomes de carbone, notamment les radicaux méthoxyméthoxy, méthoxyéthoxy, méthoxyéthoxyméthoxy, méthoxyméthoxyéthyl, méthoxyméthoxypropyl et méthoxyhexyloxy.

Par radical alkynyle, on entend un radical ayant de préférence 2 à 6 atomes de carbone, notamment un radical propargyle.

Lorsque le radical R₅ représente un atome d'halogène, celui-ci est de préférence un atome de fluor, de brome ou de chlore.

Parmi les composés de formule (I) ci-dessus, on peut notamment citer les suivants:
Acide 2-hydroxy-4-[7-(1-adamantyl)-6-benzyloxy-2-naphtyl]benzoïque.
Acide2-hydroxy-4-[7-(1-adamantyl)-6-hexyloxy-2-naphtyl]benzoïque.
Acide 4-[7-(1-adamantyl)-6-méthoxyéthoxyméthoxy-2-naphtyl]benzoïque.
Acide 5-[7-(1-adamantyl)-6-benzyloxy-2-naphtyl]-2-thiophènecarboxylique.
Acide 4-[7-(1-adamantyl)-6-benzyloxy-2-naphtyl]benzoïque.
Acide 4-[7-(1-adamantyl)-6-benzyloxycarbonyl-2-naphtyl]benzoïq ue.
Acide 2-hydroxy-4-[7-(1-adamantyl)-6-(4-fluorobenzyl)oxy-2-naphtyl]benzoïque.
Acide 6-[7-(1-adamantyl)-6-méthoxyéthoxyméthoxy-2-naphtyl]nicotinique.
Acide 4-[7-(1-adamantyl)-6-heptyloxy-2-naphtyl]benzoïque.
Acide 2-hydroxy-4-[7-(1-adamantyl)-6-méthoxyéthoxyméthoxy-2-naphtyl] benzoïque.
Acide 2-chloro-4-[7-(1-adamantyl)-6-méthoxyéthoxyméthoxy-2-naphtyl]benzoïque.
Acide 4-[7-(1-adamantyl)-6-hydroxyhexyloxy-2-naphtyl]benzoïque.
Acide 4-[7-(1-adamantyl)-6-hydroxypropyl-2-naphtyl]benzoïque.
Acide 4-[7-(1-adamantyl)-6-hydroxyoctyloxy-2-naphtyljbenzoïque.
Acide 4-[7-(1-adamantyl)-6-hydroxyéthyl-2-naphtyl]benzoïque.
Acide 4-[7-(1-adamantyl)-6-hydroxyheptyloxy-2-naphtyl]benzoïque.
Acide 4-[7-(1-adamantyl)-6-hydroxypentyloxy-2-naphtyl]benzoïque.
Acide 4-[7-(1-adamantyl)-6-(4-morpholino)éthyloxy-2-naphtyl]benzoïque.
Acide 4-[7-(1-adamantyl)-6-(1-pipéridino)éthyloxy-2-naphtyl]benzoïque.
Acide 4-[7-(1-adamantyl)-6-carbamoylpentyloxy-2-naphtyl]benzoïque.
Acide 4-[7-(1-adamantyl)-6-éthoxycarbonylpentyloxy-2-naphtyl]benzoïque
Acide 4-[7-(1-adamantyl)-6-éthoxycarbonylbutyloxy-2-naphtyl]benzoïque.
Acide 4-[7-(1-adamantyl)-6-carboxypentyloxy-2-naphtyl]benzoïque.
Acide 4-[7-(1-adamantyl)-6-carboxybutyloxy-2-naphtyl]benzoïque.
4-[7-(1-adamantyl)-6-méthoxyéthoxyméthoxy-2-naphtyl]benzèneméthanol.
4-[7-(1-adamantyl)-6-méthoxyéthoxyméthoxy-2-naphtyl]benzaldéhyde.
Morpholide de l'acide 4-[7-(1-adamantyl)-6-méthoxyéthoxyméthoxy-2-naphtyl]benzoïque.
N-éthyl-4-[7-(1-adamantyl)-6-méthoxyéthoxyméthoxy-2-naphtyl]benzamide.
4-[7-(1-adamantyl)-6-méthoxyéthoxyméthoxy-2-naphtyl]benzamide.
N-(4-hyd roxyphènyl)-4-[7-(1-adamantyl)-6-méthoxyéthoxyméthoxy-2-naphtyl]benzamide.
4-[7-(1-adamantyl)-6-méthoxyéthoxyméthoxy-2-naphtyl]benzoylpipérnzine
4-[7-(1-adamantyl)-6-méthoxyéthoxyméthoxy-2-naphtyl]benzoate de propyle.
Acétate de 4-[7-(1-adamantyl)-6-méthoxyéthoxyméthoxy-2-naphtyl]phenyle
4-[7-(1-adamantyl)-6-méthoxyéthoxyméthoxy-2-naphtyl]phenol.
Chlorhydrate de 4-[7-(1-adamantyl)-6-méthoxyéthoxyméthoxy-2-naphtyl]phenoxyéthylmorpholine.
Chlorhydrate de 4-[7-(1-adamantyl)-6-méthoxyéthoxyméthoxy-2-naphtyl]phenoxyéthylpiperid ine.
4-[7-(1-adamantyl)-6-méthoxyéthoxyméthoxy-2-naphtyl]benzoate d'hexyle.
Acide N-[[4-[7-(1-adamantyl)-6-méthoxyéthoxyméthoxy-2-naphtyl]benzoyl]]glutamique.
Acide 4-[7-(1-adamantyl)-6-méthoxyhexyloxy-2-naphtyl]benzoïque.
Acide 4-[7-(1-adamantyl)-6-méthoxymethoxypropyl-2-naphtyl]benzoïque.
Acide 4-[7-(1-adamantyl)-6-méthoxyméthoxyethyl-2-naphtyl]benzoïque.

Selon la présente invention les composés de formule (I) plus particulièrement utilisés sont ceux pour lesquels:
- R₁ est le radical -CO-R₄,
- R₂ est le radical -(X)ₙ-(CH₂)p -R₆ ou -(X)ₙ-(CH₂)_{q}-R₇
- Ar représente le radical de formule (a) ou (b).

Parmi les composés de formule (I), on préfère tout particulièrement les composés de formule (I) dans laquelle R₇ est une radical polyéther présentant un carbone en position α du carbone qui se trouve en position 6 du radical naphtyl. Ces composés s'avèrent très intéressants car ils ne semblent pas pouvoir être modifiés métaboliquement, lorsqu'ils sont administrés, en composés de type rétinoïdes induisant la différenciation de ces cellules F9 et se liant aux RARs, cette liaison étant du type agoniste.

La présente invention vise donc également ces composés spécifiques de formule (I) dans laquelle R₇ est une radical polyéther présentant un carbone en position α du carbone qui se trouve en position 6 du radical naphtyl, tels que notamment les radicaux méthoxyméthoxyéthyl et méthoxyméthoxypropyl.

Parmi ces nouveaux composés, on préfère l'acide 4-[7-(1-adamantyl)-6-méthoxymethoxypropyl-2-naphtyl]benzoïque et l'acide 4-[7-(1-adamantyl)-6-méthoxyméthoxyethyl-2-naphtyl]benzoïque.

Les composés de formule (I) peuvent être notamment obtenus:
- Soit par une réaction de couplage entre un dérivé halogéné (1) et un dérivé halogéné (2): 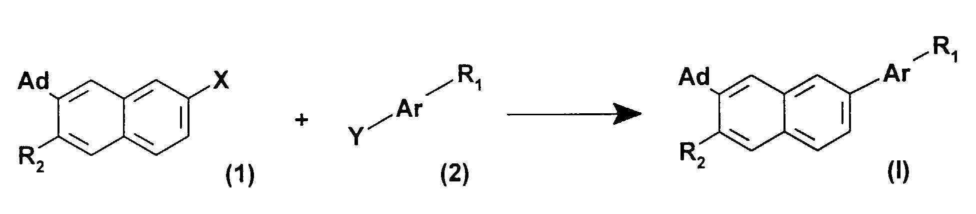 X et Y représentant un atome de chlore, de brome ou d'iode. Dans un premier temps, l'halogénure (1) est converti en un lithien ou un magnésien puis en un zincique et est couplé au dérivé (2) en présence d'un catalyseur au nickel ou au palladium, selon les conditions de couplage biaryl décrites par E, Negishi et al., J. Org. Chem (1977) 42, 1821.
- Soit par une réaction de couplage entre un acide boronique (3) et un dérivé halogéné (2):

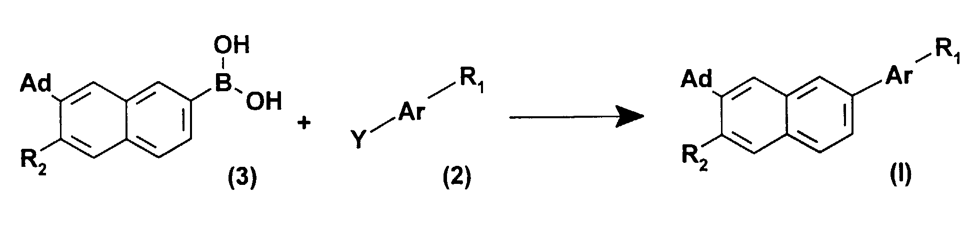

La réaction de couplage est effectuée en présence d'un catalyseur au palladium, par exemple le tétrakis(triphénylphosphine)palladium selon les conditions décrites par N. Miyaura et al., Synthetic Communications (1981) 11(7), 513-519.
Le dérivé acide boronique (3) peut être obtenu par exemple, à partir du dérivé halogéné (1) par transformation en un lithien puis réaction avec le triméthyl borate et hydrolyse.

Dans ces formules R₁, R₂, R₅ ont les mêmes significations que celles données ci-dessus pour la formule générale (I) ou en sont des dérivés convenablement protégés pour être compatibles avec les conditions de couplage.

En particulier, lorsque R₁ représente le radical -COOH, les composés sont préparés en protégeant R₁ par un groupe protecteur de type alkyle, allylique, benzylique ou tert-butylique.

Le passage à la forme libre peut être éffectué :
- dans le cas d'un groupe protecteur alkyle, au moyen de soude ou d'hydroxyde de lithium dans un solvant alcoolique tel le méthanol ou dans le THF.
- dans le cas d'un groupe protecteur allylique, au moyen d'un catalyseur tel certains complexes de métaux de transition en présence d'une amine secondaire telle la morpholine.
- dans le cas d'un groupement protecteur benzylique, par débenzylation en présence d'hydrogène au moyen d'un catalyseur tel que le palladium sur charbon.
- dans le cas d'un groupement protecteur de type tert-butylique au moyen d'iodure de triméthylsilane.

Lorsque R₂ représente les radicaux -(CH₂)ₚ-R₆, -(CH₂)_{q}-R₇, -CH=CH-(CH₂)ₛ-R₆ ou -CH=CH-(CH₂)ₜ-R₇, les composés peuvent être obtenus à partir des dérivés phénoliques correspondants (avec R₂ représentant le radical -OH) que l'on transforme en dérivés triflates puis par substitution nucléophile en présence d'un catalyseur au palladium selon les conditions générales décrites par:
- S. Cacchi et al. Tetrahedron Letters, 1986, 27, 3931-3934.
- W. J. Scott et al. J. Org. Chem. 1985, 50, 2302-2308.
- J. K. Stille et al. J. Am. Chem. Soc; 1987, 109, 5478-5486.

Les compositions cosmétique ou pharmaceutique contenant au moins un composé de formule (I) sont donc destinées au traitement de désordres ou d'affections liés à une surrégulation des récepteurs RARs et/ou à une hypervitaminose A.

Par surrégulation des récepteurs RARs, on entend selon l'invention une surexpression des récepteurs RARs et/ou une suractivité biologique des récepteurs RARs.

La suractivité biologique des récepteurs RARs peut être due à une modification chimique des récepteurs RARs, mais elle peut être due également à un facteur autre que le récepteur lui-même. Ainsi, la suractivité biologique peut être due à la surexpression d'un gène endogène ou à l'expression d'un gène exogène comprenant l'élément de réponse RARE (retinoic acid response element) sur lequel est venu se fixer un hétérodimère comprenant le récepteur RAR, ce dernier portant un ligand agoniste. A titre d'exemple de surexpression d'un gène endogène comprenant l'élément de réponse RARE, on peut citer le gène de la CRABP II (cellular retinoic acid binding protein II) dont la surexpression a été démontrée dans le psoriasis ("Overexpression of CRABP II and down-regulation of CRABP I in psoriatic skin", G. Siegenthaler et al., Dermatology 1992; 185 : 251-256). A titre d'exemple d'expression d'un gène exogène comprenant l'élément de réponse RARE, on peut citer le génome HIV-1 (virus d'immunodéficience humain) (Proc. Natl. Acad. Sci. USA, Lee et al., Vol.91, pp. 5632-5636, June 1994) ou le génome du virus de l'hépatite B ("Retinoid X receptor RXR alpha binds to and trans-activates the hepatite B virus enhancer", B. Huan et al., Proc. Natl. Acad. Sci. USA, 1992, 89 (19), p 9059-63).

Ces désordres et/ou affections liés à une surrégulation des récepteurs RARs et/ou à une hypervitaminose A se traduisent le plus souvent par une composante inflammatoire, allergique et/ou immunologique. Ils sont plus particulièrement présents dans les pathologies ou les désordres suivants :
1) les acnées vulgaires, comédoniennes, polymorphes, rosacées, les acnées nodulokystiques, conglobata, les acnées séniles, les acnées secondaires telles que l'acnée solaire, médicamenteuse ou professionnelle,
2) autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale,
4) certaines affections inflammatoires ne présentant pas de trouble de la kératinisation, telles que l'arthrite,
5) les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires,
6) autres désordres dermatologiques tels que les dermatoses bulleuses et les maladies du collagène,
7) certains troubles ophtalmologiques, notamment les cornéopathies,
8) le vieillissement de la peau, qu'il soit photo-induit ou chronologique ou les pigmentations et les kératoses actiniques, ou toutes pathologies associées au vieillissement chronologique ou actinique,
9) les stigmates de l'atrophie épidermique et/ou dermique induite par les corticostéroïdes locaux ou systémiques, ou toute autre forme d'atrophie cutanée,
10) les troubles de la cicatrisation ou les vergetures,
11) les troubles de la fonction sébacée tels que l'hyperséborrhée de l'acnée ou la séborrhée simple,
12) états cancéreux ou précancéreux,
13) affection d'origine virale au niveau cutané ou général (virus d'immunodéficience humain : HIV-1 ou virus de l'hépatite B),
14) l'alopécie,
15) affections du système cardiovasculaire telles que l'artériosclérose.

Dans le cadre de l'invention, les composés de formule (I) peuvent être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques. Par vitamines D ou leurs dérivés, on entend par exemple les dérivés de la vitamine D₂ ou D₃ et en particulier la 1,25-dihydroxyvitamine D₃. Par anti-radicaux libres, on entend par exemple l'α-tocophérol, la Super Oxyde Dismutate, l'Ubiquinol ou certains chélatants de métaux. Par α-hydroxy ou α-céto acides ou leurs dérivés, on entend par exemple les acides lactique, malique, citrique, glycolique, mandélique, tartrique, glycérique ou ascorbique ou leurs sels, amides ou esters. Enfin, par bloqueurs de canaux ioniques, on entend par exemple le Minoxidil (2,4-diamino-6-pipéridino-pyrimidine-3-oxyde) et ses dérivés.

La composition cosmétique ou pharmaceutique comprenant une quantité efficace d'au moins un composé de formule (I), l'un de ses analogues chiraux ou encore l'un de ses sels comprend un support cosmétiquement ou pharmaceutiquement acceptable et compatible avec le mode d'administration retenu.

La quantité efficace dépendant bien entendu du traitement désiré et de la nature du composé choisi est donc déterminée par l'homme du métier.

L'administration des composés selon l'invention peut être effectuée par voie entérale, parentérale, topique ou oculaire.

Par voie entérale, la composition, plus particulièrement la composition pharmaceutique, peut se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, la composition, plus particulièrement la composition pharmaceutique, peut se présenter sous forme de solutions ou de suspensions pour perfusion ou pour injection.

Les composés selon l'invention sont généralement administrés à une dose journalière d'environ 0,01 mg/kg à 100 mg/kg en poids corporel, et ceci à raison de 1 à 3 prises.

Par voie topique, la composition est plus particulièrement destinée au traitement de la peau et des muqueuses et peut alors se présenter sous forme d'onguents, de crêmes, de laits, de pommades, de poudres, de tampons imbibés, de solutions, de gels, de sprays, de lotions, de suspensions ou de shampooings. Il peut également se présenter sous forme de microsphères ou nanosphères ou vésicules lipidiques ou polymériques ou de patches polymériques et d'hydrogels permettant une libération contrôlée. Cette composition par voie topique peut par ailleurs se présenter soit sous forme anhydre, soit sous une forme aqueuse.

Par voie oculaire, ce sont principalement des collyres.

Cette composition à usage topique ou oculaire contient au moins un composé de formule (I) telle que définie ci-dessus, ou l'un de ses analogues chiraux ou encore l'un de ses sels, à une concentration de préférence comprise entre 0,001% et 5% en poids par rapport au poids total de la composition.

La composition selon l'invention peut en outre contenir des additifs inertes ou même pharmacodynamiquement ou cosmétiquement actifs ou des combinaisons de ces additifs, et notamment : des agents mouillants ; des agents dépigmentants tels que l'hydroquinone, l'acide azélaïque, l'acide caféïque ou l'acide kojique ; des émollients ; des agents hydratants comme le glycérol, le PEG 400, la thiamorpholinone, et ses dérivés ou bien encore l'urée ; des agents antiséborrhéiques ou antiacnéiques, tels que la S-carboxyméthylcystéine, la S-benzyl-cystéamine, leurs sels ou leurs dérivés, ou le péroxyde de benzoyle ; des antibiotiques comme l'érythromycine et ses esters, la néomycine, la clindamycine et ses esters, les tétracyclines ; des agents antifongiques tels que le kétoconazole ou les polyméthylène-4,5 isothiazolidones-3 ; des agents favorisant la repousse des cheveux, comme le Minoxidil (2,4-diamino-6-pipéridinopyrimidine-3-oxyde) et ses dérivés, le Diazoxide (7-chloro 3- méthyl 1,2,4-benzothiadiazine 1,1-dioxyde) et le Phénytoïn (5,4-diphényl-imidazolidine 2,4-dione) ; des agents anti-inflammatoires non stéroïdiens ; des caroténoïdes et, notamment, le β-carotène; des agents anti-psoriatiques tels que l'anthraline et ses dérivés ; et enfin les acides eicosa-5,8,11,14-tétraenoïque et eicosa-5,8,11-trienoïque, leurs esters et amides.

La composition peut également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluène.

On va maitenant donner, à titre d'illustration et sans aucun caractère limitatif, plusieurs exemples d'obtention de composés actifs de formule (I), ainsi que diverses formulations concrètes à base de tels composés.

### EXEMPLE 1

### Acide 2-hydroxy-4-[7-(1-adamantyl)-6-benzyloxy-2-naphthyl]benzoïque.

### (a) 7-(1-adamantyl)-6-benzyloxy-2-bromonaphtalène.

Dans un tricol on introduit 1,26 g (42 mmoles) d'hydrure de sodium (80% dans l'huile) et 50 ml de DMF. On ajoute goutte à goutte une solution de 12,5 g (35 mmoles) de 7-(1-adamantyl)-6-hydroxy-2-bromonaphtalène dans 100 ml de DMF et agite jusqu'à cessation du dégagement gazeux. On ajoute ensuite 5 ml (42 mmoles) de bromure de benzyle et agite à température ambiante deux heures. On verse le milieu réactionnel dans l'eau, extrait avec de l'éther éthylique, décante la phase organique, sèche sur sulfate de magnésium, évapore. On reprend le résidu obtenu dans l'éthanol, chauffe à reflux, refroidit, filtre et sèche. On recueille 12,5 g (80%) du produit attendu de point de fusion 150-1°C.

### (b) acide 7-(1-adamantyl)-6-benzyloxy-2-naphtylboronique.

Dans un tricol et sous courant d'azote, on introduit 3 g (6,7 mmoles) de 7-(1-adamantyl)-6-benzyloxy-2-bromonaphtalène et 50 ml de THF. A -78°C, on ajoute goutte à goutte 3,2 ml (8 mmoles) de n-butyllithium (2,5M dans l'hexane) et agite 15', à cette même température on ajoute 2,1 g (20 mmoles) de triméthylborate et agite pendant 2 heures. A -50°C on ajoute 23 ml d'acide chlorhydrique (1N) et laisse remonter à température ambiante. On extrait le milieu réactionnel avec de l'éther éthylique, décante la phase organique, sèche sur sulfate de magnésium, évapore. Le résidu obtenu est trituré dans l'heptane, filtré, séché. On recueille 2,8 g (100%) d'acide boronique attendu qui est utilisé tel quel pour la suite de la synthèse.

### (c) 2-hydroxy-4-[7-(1-adamantyl)-6-benzyloxy-2-naphtyl]benzoate de méthyle.

Dans un tricol et sous courant d'azote, on introduit 300 mg (8,8 mmoles) de tétrakis(triphénylphosphine)palladium(0), 50 ml de toluène et 2,46 g (8,8 mmoles) de 2-hydroxy-4-iodobenzoate de méthyle et agite à température ambiante 20'. On ajoute ensuite 5,52 g (13,4 mmoles) d'acide 7-(1-adamantyl)-6-benzyloxy-2-naphtylboronique et 8,8 ml d'une solution aqueuse de carbonate de potassium (2N) et chauffe à reflux pendant 8 heures. On évapore à sec le milieu réactionnel, reprend par l'eau et l'éther éthylique, décante la phase organique, sèche sur sulfate de magnésium, évapore. Le résidu est purifié par chromatographie sur colonne de silice éluée avec un mélange d'acétate d'éthyle et d'heptane (10-90). On obtient 1,65 g (36%) de 2-hydroxy-4-[7-(1-adamantyl)-6-benzyloxy-2-naphtyl]benzoate de méthyle.

### (d) acide 2-hydroxy-4-[7-(1-adamantyl)-6-benzyloxy-2-naphtyl]benzoïque.

Dans un ballon, on introduit 930 mg (1,8 mmole) de 2-hydroxy-4-[7-(1-adamantyl)-6-benzyloxy-2-naphtyl]benzoate de méthyle et 100 ml d'une solution de soude méthanolique 2N et chauffe à reflux pendant une heure. On évapore à sec le milieu réactionnel, reprend le résidu par l'eau, acidifie à pH 1 avec de l'acide chlorhydrique concentré et filtre le solide. On triture le solide obtenu dans l'acétate d'éthyle, filtre, sèche. On recueille 710 mg (79%) de l'acide attendu de point de fusion 263-4°C.

### EXEMPLE 2

### Acide 2-hydroxy-4-[7-(1-adamantyl)-6-hexyloxy-2-naphtyl]benzoïque.

### (a) 2-hydroxy4-[7-(1-adamantyl)-6-hexyloxy-2-naphtyl]benzoate de méthyle.

De manière analogue à l'exemple 1(a) par réaction de 430 mg (1 mmole) de 2-hydroxy-4-[7-(1-adamantyl)-6-hydroxy-2-naphtyl]benzoate de méthyle avec 180 µl (1,2 mmole) de 6-iodohexane, on obtient 280 mg (55%) de 2-hydroxy-4-[7-(1-adamantyl)-6-hexyloxy-2-naphtyl]benzoate de méthyle.

### (b) acide 2-hydroxy4-[7-(1-adamantyl)-6-hexyloxy-2-naphtyl]benzoïque.

De manière analogue à l'exemple 1(d) à partir de 100 mg (0,2 mmole) de 2-hydroxy4-[7-(1-adamantyl)-6-hexyloxy-2-naphtyl]benzoate de méthyle, on obtient 90 mg (92%) de l'acide attendu de point de fusion 281-3°C.

### EXEMPLE 3

### Acide 4-[7-(1-adamantyl)-6-méthoxyéthoxyméthoxy-2-naphtyl]benzoïque.

### (a) 4-[7-(1-adamantyl)-6-benzyloxy-2-naphtyl]benzoate de méthyle.

De manière analogue à l'exemple 1(c) par réaction de 2,8 g (6,7 mmoles) d'acide 7-(1-adamantyl)-6-benzyloxy-2-naphtylboronique avec 950 mg (4,4 mmoles) de 4-bromobenzoate de méthyle, on obtient 1,6 g (72%) du produit attendu.

### (b) 4-[7-(1-adamantyl)-6-hydroxy-2-naphtyl]benzoate de méthyle.

Dans un réacteur on introduit 1,38 g (2,75 mmoles) de 4-[7-(1-adamantyl)-6-benzyloxy-2-naphtyl]benzoate de méthyle, 450 mg de palladium sur charbon (10%) et 50 ml de dioxanne. On ajoute 5 gouttes d'acide acétique et hydrogène à 50°C et sous une pression de 6,5 bars d'hydrogène pendant 4 heures. On filtre le catalyseur, lave avec deux fois 20 ml de dioxanne, évapore les filtrats. Le résidu obtenu est purifié par chromatographie sur colonne de silice éluée avec un mélange de dichlorométhane et d'hexane (50-50). On recueille 980 mg (86%) de 4-[7-(1-adamantyl)-6-hydroxy-2-naphtyl]benzoate de méthyle sous forme d'une huile.

### (c) 4-[7-(1-adamantyl)-6-méthoxyéthoxyméthoxy-2-naphtyl]benzoate de méthyle.

De manière analogue à l'exemple 1(a) par réaction de 980 mg (2,4 mmoles) de 4-[7-(1-adamantyl)-6-hydroxy-2-naphtyl]benzoate de méthyle avec 330 µl (28,6 mmoles) de chlorure de méthoxyéthoxyméthane, on obtient 650 mg (55%) du produit attendu sous forme d'une huile.

### (d) acide 4-[7-(1-adamantyl)-6-méthoxyéthoxyméthoxy-2-naphtyl]benzoïque.

De manière analogue à l'exemple 1(d) à partir de 650 mg (1,3 mmole) de 4-[7-(1-adamantyl)-6-méthoxyéthoxyméthoxy-2-naphtyl]benzoate de méthyle, on obtient 580 mg (92%) de l'acide attendu de point de fusion 234-6°C.

### EXEMPLE 4

### Acide 5-[7-(1-adamantyl)-6-benzyloxy-2-naphthyl]-2-thiophènecarboxylique.

### (a) 5-[7-(1-adamantyl)-6-benzyloxy-2-naphtyl]-2-thiophènecarboxylate de méthyle.

De manière analogue à l'exemple 1(c) par réaction de 1,5 g (3,6 mmoles) d'acide 7-(1-adamantyl)-6-benzyloxy-2-naphtylboronique avec 400 mg (1,8 mmole) de 5-bromo-2-thiophènecarboxylate de méthyle, on obtient 600 mg (65%) du produit attendu de point de fusion 170-1°C.

### (b) acide5-[7-(1-adamantyl)-6-benzyloxy-2-naphtyl]-2-thiophènecarboxylique.

De manière analogue à l'exemple 1(d) à partir de 600 mg (1,2 mmole) de 5-[7-(1-adamantyl)-6-benzyloxy-2-naphtyl]-2-thiophènecarboxylate de méthyle, on obtient 460 mg (79%) d'acide attendu de point de fusion 271-3°C.

### EXEMPLE 5

### Acide 4-[7-(1-adamantyl)-6-benzyloxy-2-naphyl]benzoïque.

### (a) 4-[7-(1-adamantyl)-6-benzyloxy-2-naphtyl]benzoate de méthyle.

De manière analogue à l'exemple 1(c) par réaction de 1,5 g (3,6 mmoles) d'acide 7-(1-adamantyl)-6-benzyloxy-2-naphtylboronique avec 500 mg (1,9 mmole) de 4-iodobenzoate de méthyle, on obtient 320 mg (33%) du produit attendu de point de fusion 170-3°C.

### (b) acide 4-[7-(1-adamantyl)-6-benzyloxy-2-naphtyl]benzoïque.

De manière analogue à l'exemple 1(d) à partir de 320 mg (0,6 mmole) de 4-[7-(1-adamantyl)-6-benzyloxy-2-naphtyl]benzoate de méthyle, on obtient 195 mg (63%) d'acide attendu de point de fusion 305-10°C.

### EXEMPLE 6

### Acide 4-[7-(adamantyl)-6-benzyloxycarbonyl-2-naphtyl]benzoïque.

### (a) 4-[7-(1-adamantyl)-6-trifluorométhylsulfonyloxy-2-naphtyl]benzoate de méthyle.

A une solution refroidie à -78°C de 5,5 g (13,3 mmoles) de 4-[7-(1-adamantyl)-6-hydroxy-2-naphtyl]benzoate de méthyle 3,2 ml (40 mmoles) de pyridine et 162 mg de 4-diméthylaminopyridine dans 100 ml de dichlorométhane, on ajoute goutte à goutte 2,7 ml (16 mmoles) d'anhydride trifluorométhanesulfonique et agite à température ambiante pendant 12 heures. On verse le milieu réactionnel dans l'eau glacée, extrait avec de l'éther éthylique, décante la phase organique, lave avec une solution saturée de chlorure de sodium, sèche sur sulfate de magnésium, évapore. Le résidu obtenu est purifié par chromatographie sur colonne de silice éluée avec un mélange d'acétate d'éthyle et d'heptane (10-90). On recueille 1,94 g (27%) de 4-[7-(1-adamantyl)-6-trifluorométhylsulfonyl oxy-2-naphtyl]benzoate de méthyle de point de fusion 226-7°C.

### (b) 4-[7-(1-adamantyl)-6-benzyloxycarbonyl-2-naphtyl]benzoate de méthyle.

Dans un réacteur, on introduit sucessivement une solution de 1,91 g (3,5 mmoles) de 4-[7-(1-adamantyl)-6-trifluorométhylsulfonyloxy-2-naphtyl]benzoate de méthyle dans 50 ml de DMF, 980 µl (7 mmoles) de triéthylamine, 39 mg d'acétate de palladium, 195 mg (0,35 mmole) de 1,1'-bis(diphénylphosphino)ferrocène et 3,65 ml (35,1 mmoles) d'alcool benzylique. On chauffe à 80°C sous une pression de 2,5 bars d'oxyde de carbone pendant 12 heures. On verse le milieu réactionnel dans une solution saturée de chlorure de sodium, extrait avec de l'éther éthylique, décante la phase organique, sèche sur sulfate de magnésium, évapore. Le résidu obtenu est purifié par chromatographie sur colonne de silice éluée avec un mélange d'acétate d'éthyle et d'heptane (15-85). Après évaporation des solvants, on recueille 720 mg (40%) du produit attendu de point de fusion 143-4°C.

### (c) Acide 4-[7-(1-adamantyl)-6-benzyloxycarbonyl-2-naphtyl]benzoïque.

De manière analogue à l'exemple 1(d) à partir de 260 mg (0,5 mmole) de 4-[7-(1-adamantyl)-6-benzyloxycarbonyl-2-naphtyl]benzoate de méthyle, on obtient 200 mg (79%) d'acide attendu de point de fusion 224-6°C.

### EXEMPLE 7

### Acide 2-hydroxy-4-[7-(1-adamantyl)-6-(4-fluorobenzyl)oxy-2-naphtyl] benzoïque.

### (a) 7-(1-adamantyl)-6-(4-fluorobenzyl)oxy-2-bromonaphtalène.

De manière analogue à l'exemple 1(a) par réaction de 1,1 g (3 mmoles) de 7-(1-adamantyl)-6-hydroxy-2-bromonaphtalène avec 420 µl (3,3 mmoles) de bromure de 4-fluorobenzyle, on obtient 1,2 g (86%) du produit attendu sous forme d'une huile incolore.

### (b) acide 7-(1-adamantyl)-6-(4-fluorobenzyl)oxy-2-naphtylboronique.

De manière analogue à l'exemple 1(b) à partir de 1,14 g (2,45 mmoles) de 7-(1-adamantyl)-6-(4-fluorobenzyl)oxy-2-bromonaphtalène, on obtient 560 mg (57%) d'acide boronique attendu.

### (c) 2-hydroxy-4-[7-(1-adamantyl)-6-(4-fluorobenzyl)oxy-2-naphtyl]benzoate de méthyle.

De manière analogue à l'exemple 1(c) par réaction de 560 mg (1,41 mmole) d'acide 7-(1-adamantyl)-6-(4-fluorobenzyl)oxy-2-naphtylboronique avec 330 mg (1,17 mmole) de 2-hydroxy-4-iodobenzoate de méthyle, on obtient 490 mg (78%) de l'ester attendu de point de fusion 189-91°C.

### (d) acide 2-hyd roxy-4-[7-(1adamantyl)-6-(4-fluorobenzyl)oxy-2-naphtyl]benzoïque.

De manière analogue à l'exemple 1(d) à partir de 490 mg (0,91 mmole) de 2-hydroxy-4-[7-(1-adamantyl)-6-(4-fluorobenzyl)oxy-2-naphtyl]benzoate de méthyle, on obtient 440 mg (92%) d'acide attendu de point de fusion 240-1°C.

### EXEMPLE 8

### Acide 6-[7-(1-adamantyl)-6-méthoxyéthoxyméthoxy-2-naphtyl]nicotinique.

### (a) 3-(1-adamantyl)-6-bromo-2-naphtol.

Dans un ballon, on introduit 56 g (0,25 mole) de 6-bromo-2-naphtol, 38,2 g (0,25 mole) de 1-adamantanol et 500 ml d'un mélange de dichlorométhane et d'heptane (40-60). On ajoute 15 ml d'acide sulfurique concentré et agite à température ambiante pendant 48 heures. On filtre le solide, lave avec l'heptane (3x100 ml), dissout le solide dans l'éther éthylique, lave à l'eau, décante la phase organique, sèche sur sulfate de magnésium, évapore. On recueille 60,1 g (67%) du produit attendu de point de fusion 215-6°C.

### (b) 7-(1-adamantyl)-6-méthoxyéthoxyméthoxy-2-bromonaphtalène.

Dans un tricol et sous courant d'azote, on introduit 17,85 g (0,05 mole) de 3-(1-adamantyl)-6-bromo-2-naphtol et 200 ml de DMF. On ajoute par petites quantités 1,8 g (0,06 mole) d'hydrure de sodium (80% dans l'huile) et agite jusqu'à cessation du dégagement gazeux. On ajoute ensuite 6,9 ml (0,06 mole) de chlorure de méthoxyéthoxyméthane et agite à température ambiante pendant 2 heures. On verse le milieu réactionnel dans l'eau glacée, extrait avec de l'éther éthylique, décante la phase organique, sèche sur sulfate de magnésium, évapore. Le résidu obtenu est purifié par chromatographie sur colonne de silice éluée avec un mélange de dichlorométhane et d'heptane (40-60). On recueille 19,5 g (87%) du produit attendu de point de fusion 99-100°C.

### (c) Acide 7-(1-adamantyl)-6-méthoxyéthoxyméthoxy-2-naphtylboronique.

De manière analogue à l'exemple 1(b) à partir de 61,9 g (0,139 mole) de 7-(1-adamantyl)-6-méthoxyéthoxyméthoxy-2-bromonaphtalène, on obtient 54 g (95%) d'acide 7-(1-adamantyl)-6-méthoxyéthoxyméthoxy-2-naphtylboronique de point de fusion 172-4°C.

### (d) 6-[7-(1-adamantyl)-6-méthoxyéthoxyméthoxy-2-naphtyl]nicotinate de méthyle.

De manière analogue à l'exemple 1(c) par réaction de 1,3 g (3,1 mmoles) d'acide 7-(1-adamantyl)-6-méthoxyéthoxyméthoxy-2-naphtylboronique avec 790 mg (3 mmoles) de 6-iodonicotinate de méthyle, on obtient 860 mg (57%) de l'ester attendu de point de fusion 166-7°C.

### (e) acide 6-[7-(1-adamantyl)-6-méthoxyéthoxyméthoxy-2-naphtyl]nicotinique.

De manière analogue à l'exemple 1(d) à partir de 853 mg (1,7 mmole) de l'ester méthylique précédent, on obtient 790 mg (95%) d'acide attendu de point de fusion 247-8°C.

### EXEMPLE 9

### Acide 4-[7-(1-adamantyl)-6-heptyloxy-2-naphyl]benzoïque.

### (a) 7-(1-adamantyl)-6-heptyloxy-2-bromonaphtalène.

De manière analogue à l'exemple 8(b) par réaction de 3,4 g (9,5 mmoles) de 3-(1-adamantyl)-6-bromo-2-naphtol avec 2,05 g (11,4 mmoles) de 1-bromoheptane, on obtient 3,4 g (79%) de 7-(1-adamantyl)-6-heptyloxy-2-bromonaphtalène.

### (b) Acide 7-(1-adamantyl)-6-heptyloxy-2-naphtylboronique.

De manière analogue à l'exemple 1(b) à partir de 3,5 g (17,7 mmoles) de 7-(1-adamantyl)-6-heptyloxy-2-bromonaphtalène, on obtient 1,67 g (51%) d'acide 7-(1-adamantyl)-6-heptyloxy-2-naphtylboronique.

### (c) 4-[7-(1-adamantyl)-6-heptyloxy-2-naphtyl]benzoate de méthyle.

De manière analogue à l'exemple 1(c) par réaction de 1,6 g (3,8 mmoles) d'acide 7-(1-adamantyl)-6-heptyloxy-2-naphtylboronique avec 830 mg (3,17 mmoles) de 4-iodobenzoate de méthyle, on obtient 460 mg (29%) de l'ester méthylique attendu.

### (d) Acide 4-[7-(1-adamantyl)-6-heptyloxy-2-naphtyl]benzoïque.

De manière analogue à l'exemple 1(d) à partir de 330 mg (0,65 mmole) de 4-[7-(1-adamantyl)-6-heptyloxy-2-naphtyl]benzoate de méthyle, on obtient 260 mg (81%) d'acide attendu de point de fusion 266-7°C.

### EXEMPLE 10

### Acide 2-hydroxy-4-[7-(1-adamantyl)-6-méthoxyéthoxyméthoxy-2-naphtyl] benzoïque.

### (a) 2-hydroxy-4-[7-(1-adamantyl)-6-méthoxyéthoxyméthoxy-2-naphtyl]benzoate de méthyle.

De manière analogue à l'exemple 1(c) par réaction de 2,25 g (5,5 mmoles) d'acide 7-(1-adamantyl)-6-méthoxyéthoxyméthoxy-2-naphtylboronique avec 1,39 g (5 mmoles) de 2-hydroxy-4-iodobenzoate de méthyle, on obtient 2,1 g (81%) de l'ester attendu de point de fusion 101-2°C.

### (b) acide 2-hydroxy-4-[7-(1-adamantyl)-6-méthoxyéthoxyméthoxy-2-naphtyl] benzoïque.

De manière analogue à l'exemple 1(d) à partir de 2,08 g (4 mmoles) de l'ester méthylique précédent, on obtient 1,72 g (86%) d'acide attendu de point de fusion 225-6°C.

### EXEMPLE 11

### Acide 2-chloro-4-[7-(1-adamantyl)-6-méthoxyéthoxyméthoxy-2-naphtyl]benzoïque.

### (a) 2-chloro-4-[7-(1-adamantyl)-6-méthoxyéthoxyméthoxy-2-naphtyl]benzoate de méthyle.

De manière analogue à l'exemple 1(c) par réaction de 2,25 g (5,5 mmoles) d'acide 7-(1-adamantyl)-6-méthoxyéthoxyméthoxy-2-naphtylboronique avec 1,48 g (5 mmoles) de 2-chloro-4-iodobenzoate de méthyle, on obtient 2,32 g (87%) de l'ester attendu de point de fusion 112-3°C.

### (b) acide 2-chloro-4-[7-(1-adamantyl)-6-méthoxyéthoxyméthoxy-2-naphtyl] benzoïque.

De manière analogue à l'exemple 1(d) à partir de 2,31 g (4,3 mmoles) de l'ester méthylique précédent, on obtient 1,97 g (88%) d'acide attendu de point de fusion 190-2°C.

### EXEMPLE 12

### Acide 4-[-7-(1-adamantyl)-6-hydroxyhexyloxy-2-naphtyl]benzoïque.

### (a) 4-[7-(1-adamantyl)-6-hydroxyhexyloxy-2-naphtyl]benzoate de méthyle.

Dans un ballon et sous courant d'azote, on introduit 1,62 g (40 mmoles) de 4-[7-(1-adamantyl)-6-hydroxy-2-naphtyl]benzoate de méthyle, 830 mg de carbonate de potassium et 60 ml de méthyléthylcétone. On ajoute 1,09 g (60 mmoles) de 6-bromo-1-hexanol et chauffe à reflux pendant douze heures. On verse le mileu réactionnel dans l'acide chlorhydrique 1 N, extrait avec de l'éther éthylique, décante la phase organique, sèche sur sulfate de magnésium, évapore. On purifie le résidu obtenu par chromatographie sur colonne de silice éluée avec du dichlorométhane. Après évaporation des solvants, on recueille 1,58 g (77%) de l'ester méthylique attendu de point de fusion 153-5°C.

### (b) acide 4-[7-(1-adamantyl)-6-hydroxyhexyloxy-2-naphtyl]benzoïque.

De manière analogue à l'exemple 1(d) à partir de 700 mg (1,36 mmole) de 4-[7-(1-adamantyl)-6-hydroxyhexyloxy-2-naphtyl]benzoate de méthyle, on obtient 417 mg (61%) de l'acide attendu de point de fusion 254-6°C.

### EXEMPLE 13

### Acide 4-[7-(1-adamantyl)-6-hydroxypropyl-2-naphyl]benzoïque.

### (a) 4-[7-(1-adamantyl)-6-allyl-2-naphtyl]benzoate de méthyle.

Dans un tricol et sous courant d'azote, on introduit 4 g (7,34 mmoles) de 4-[7-(1-adamantyl)-6-trifluorométhylsulfonyloxy-2-naphtyl]benzoate de méthyle 2,30 ml (7,9 mmoles) d'allyltributylétain, 630 mg de chlorure de lithium et 40 ml de DMF. On agite à température ambiante trente minutes, ajoute 104 mg (0,146 mmoles) de chlorure de bis(triphénylphosphine)palladium(ll) et chauffe à 100°C pendant trois heures. On verse le milieu réactionnel dans l'eau, extrait avec de l'éther éthylique, décante la phase organique, sèche sur sulfate de magnésium, évapore. On purifie le résidu obtenu par chromatographie sur colonne de silice éluée avec un mélange d'heptane et de dichlorométhane (50-50). On recueille 1,7 g (53%) du produit attendu de point de fusion 171-3°C.

### (b) 4-[7-(1-adamantyl)-6-hydroxypropyl-2-naphtyl]benzoate de méthyle.

Dans un ballon et sous courant d'azote, on introduit 1,7 g (3,9 mmoles) de 4-[7-(1-adamantyl)-6-allyl-2-naphtyl]benzoate de méthyle et 40 ml de THF. A 0°C on ajoute goutte à goutte une solution de 23,5 ml (11,7 mmoles) de 9-borabicyclo(3,3,1)nonane (0,5 M dans le THF) et agite une heure à température ambiante. On ajoute ensuite successivement et à 0°C 12 ml (12 mmoles) d'une solution d'hydroxyde de sodium (1M) et 10 ml d'une solution de peroxyde d'hydrogène à 30% et agite à température ambiante une heure. On verse le milieu réactionnel dans l'eau, extrait avec de l'éther éthylique, décante la phase organique, sèche sur sulfate de magnésium, évapore. On purifie la poudre blanche obtenue par chromatographie sur colonne de silice éluéee avec du dichlorométhane. On recueille 1,67 g (94%) de produit attendu de point de fusion 184-6°C.

### (c) acide 4-[7-(1-adamantyl)-6-hydroxypropyl-2-naphtyl]benzoïque.

De manière analogue à l'exemple 1(d) à partir de 500 mg (1,1 mmole) de l'ester précédent, on recueille 417 mg (86%) d'acide 4-[7-(1-adamantyl)-6-hydroxypropyl-2-naphtyl]benzoïque de point de fusion 268-9°C.

### EXEMPLE 14

### Acide 4-[7-(1-adamantyl)-6-hydroxyoctyloxy-2-naphtyl]benzoïque.

### (a) 4-[7-(1-adamantyl)-6-hydroxyoctyloxy-2-naphtyl]benzoate de méthyle.

De manière analogue à l'exemple 12(a) par réaction de 1,5 g (3,6 mmoles) de 4-[7-(1-adamantyl)-6-hydroxy-2-naphtyl]benzoate de méthyle avec 930 µl (5,45 mmoles) de 1-bromo-8-octanol, on obtient 800 mg (41%) de produit attendu de point de fusion 120-1°C.

### (b) acide 4-[7-(1-adamantyl)-6-hydroxyoctyloxy-2-naphtyl]benzoïque.

De manière analogue à l'exemple 1(d) à partir de 630 mg (1,17 mmole) de l'ester précédent on obtient 487 mg (79%) d'acide 4-[7-(1-adamantyl)-6-hydroxyoctyloxy-2-naphtyl]benzoïque de point de fusion 242-3°C.

### EXEMPLE 15

### Acide 4-[7-(1-adamantyl)-6-hydroxyéthyl-2-naphtyl]benzoïque.

### (a) 4-[7-(1-adamantyl)-6-vinyl-2-naphtyl]benzoate de méthyle.

De manière analogue à l'exemple 13(a) par réaction de 4,82 g (8,86 mmoles) de 4-[7-(1-adamantyl)-6-trifluorométhylsulfonyloxy-2-naphtyl]benzoate de méthyle avec 3,90 ml (13,3 mmoles) de vinyltributylétain, on obtient 805 mg (21,5%) de 4-[7-(1-adamantyl)-6-vinyl-2-naphtyl]benzoate de méthyle de point de fusion 221-2°C.

### (b) 4-[7-(1-adamantyl)-6-hydroxyéthyl-2-naphtyl]benzoate de méthyle.

De manière analogue à l'exemple 13(b) à partir de 794 mg (1,88 mmoles) de 4-[7-(1-adamantyl)-6-vinyl-2-naphtyl]benzoate de méthyle, on obtient 430 mg (54%) de l'alcool attendu de point de fusion 168-70°C.

### (c) acide 4-[7-(1-adamantyl)-6-hydroxyéthyl-2-naphyl]benzoïque.

De manière analogue à l'exemple 1(d) à partir de 108 mg (0,25 mmole) de l'ester précédent, on recueille 60 mg (38%) d'acide 4-[7-(1-adamantyl)-6-hydroxyéthyl-2-naphtyl]benzoïque de point de fusion 276-8°C.

### EXEMPLE 16

### Acide 4-[7-(1-adamantyl)-6-hydroxyheptyloxy-2-naphtyl]benzoïque.

### (a) 4-[7-(1-adamantyl)-6-hydroxyheptyloxy-2-naphtyl]benzoate de méthyle.

De manière analogue à l'exemple 12(a) par réaction de 1,5 g (3,6 mmoles) de 4-[7-(1-adamantyl)-6-hydroxy-2-naphtyl]benzoate de méthyle avec 840 µl (5,45 mmoles) de 1-bromo-7-heptanol, on obtient 1 g (52%) de produit attendu de point de fusion 150-1°C.

### (b) acide 4-[7-(1-adamantyl)-6-hydroxyheptyloxy-2-naphtyl]benzoïque.

De manière analogue à l'exemple 1(d) à partir de 1 g (1,9 mmole) de l'ester méthylique précédent, on obtient 830 mg (86%) d'acide 4-[7-(1-adamantyl)-6-hydroxyheptyloxy-2-naphtyl]benzoïque de point de fusion 227-8°C.

### EXEMPLE 17

### Acide 4-[7-(1-adamantyl)-6-hydroxypentyloxy-2-naphtyl]benzoïque.

### (a) 4-[7-(1-adamantyl)-6-acétoxypentyloxy-2-naphtyl]benzoate de méthyle.

De manière analogue à l'exemple 12(a) par réaction de 1 g (2,4 mmoles) de 4-[7-(1-adamantyl)-6-hydroxy-2-naphtyl]benzoate de méthyle avec 760 mg (3,6 mmoles) d'acétate de 5-bromopentyle, on obtient 1,3 g (100%) de l'ester méthylique attendu de point de fusion 132-3°C.

### (b) acide 4-[7-(1-adamantyl)-6-hydroxypentyloxy-2-naphtyl]benzoïque.

De manière analogue à l'exemple 1(d) à partir de 1,3 g (2,4 mmoles) de l'ester précédent, on obtient 1 g (79%) de l'acide attendu de point de fusion 271-2°C.

### EXEMPLE 18

### Acide 4-[7-(1-adamantyl)-6-(4-morpholino)éthyloxy-2-naphtyl]benzoïque.

### (a) 4-[7-(1-adamantyl)-6-(4-morpholino)éthyloxy-2-naphtyl]benzoate de méthyle.

De manière analogue à l'exemple 12(a) par réaction de 1,5 g (3,6 mmoles) de 4-[7-(1-adamantyl)-6-hydroxy-2-naphtyl]benzoate de méthyle avec 5,45 g (5,45 mmoles) de chlorhydrate de 4-(2-chloroéthyl)morpholine, on obtient 1,52 g (88%) de l'ester méthylique attendu de point de fusion 198-9°C.

### (b) acide 4-[7-(1-adamantyl)-6-(4-morpholino)éthyloxy-2-naphtyl]benzoïque.

De manière analogue à l'exemple 1(d) à partir de 1,45 g (2,76 mmoles) de l'ester précédent, on obtient 956 mg (68%) de l'acide attendu de point de fusion 280°C avec décomposition.

### EXEMPLE 19

### Acide 4-[7-(1-adamantyl)-6-(1-pipéridino)éthyloxy-2-naphtyl]benzoïque.

### (a) 4-[7-(1-adamantyl)-6-(1-pipéridino)éthyloxy-2-naphtyl]benzoate de méthyle.

De manière analogue à l'exemple 12(a) par réaction de 1,5 g (3,6 mmoles) de 4-[7-(1-adamantyl)-6-hydroxy-2-naphtyl]benzoate de méthyle avec 1 g (5,45 mmoles) de chlorhydrate de 1-(2-chloroéthyl)pipéridine on obtient 1,46 g (77%) de l'ester méthylique attendu de point de fusion 240-1°C.

### (b) acide 4-[7-(1-adamantyl)-6-(1-pipéridino)éthyloxy-2-naphtyl]benzoïque.

De manière analogue à l'exemple 1(d) à partir de 1,25 g (2,3 mmoles) de l'ester précédent, on obtient 640 mg (53%) de l'acide attendu de point de fusion 250°C avec décomposition.

### EXEMPLE 20

### Acide 4-[7-(1-adamantyl)-6-carbamoylpentyloxy-2-naphtyl]benzoïque.

### (a) 4-[7-(1-adamantyl)-6-carbamoylpentyloxy-2-naphtyl]benzoate de méthyle

De manière analogue à l'exemple 12(a) par réaction de 640 mg (1,55 mmoles) de 4-[7-(1-adamantyl)-6-hydroxy-2-naphtyl]benzoate de méthyle avec 450 mg (2,32 mmoles) de 6-bromohexanamide on obtient 520 mg (65%) de produit attendu de point de fusion 209-10°C.

### (b) acide 4-[7-(1-adamantyl)-6-carbamoylpentyloxy-2-naphtyl]benzoïque.

De manière analogue à l'exemple 1(d) à partir de 400 mg (0,76 mmole) de l'ester précédent, on obtient 350 mg (90%) de l'acide attendu de point de fusion 270-1°C.

### EXEMPLE 21

### Acide 4-[7-(1-adamantyl)-6-éthoxycarbonylpentyloxy-2-naphylbenzoïque

### (a) 4-[7-(1-adamantyl)-6-éthoxycarbonylpentyloxy-2-naphtyl]benzoate d'allyle.

De manière analogue à l'exemple 12(a) par réaction de 3 g (6,8 mmoles) de 4-[7-(1-adamantyl)-6-hydroxy-2-naphtyl]benzoate d'allyle avec 2,3 g (10,2 mmoles) de 6-bromohexanoate d'éthyle on obtient 2,15 g (55%) de produit attendu de point de fusion 116-7°C.

### (b) acide 4-[7-(1-adamantyl)-6-éthoxycarbonylpentyloxy-2-naphtyl]benzoïque.

Dans un tricol et sous courant d'azote, on introduit 1,5 g (2,58 mmoles) de 4-[7-(1-adamantyl)-6-éthoxycarbonylpentyloxy-2-naphtyl]benzoate d'allyle, 50 ml de THF et 90 mg (0,08 mmole) de tétrakis(triphénylphosphine)palladium(0). On ajoute goutte à goutte 1,13 ml (13 mmoles) de morpholine et agite à température ambiante deux heures. On évapore à sec le milieu réactionnel, reprend par l'eau, acidifie à pH 1 avec de l'acide chlorhydrique, extrait avec de l'éther éthylique, décante la phase organique, sèche sur sulfate de magnésium, évapore. On triture le solide dans l'heptane, filtre, sèche. On recueille 1 g (72%) de l'acide attendu de point de fusion 270-1°C.

### EXEMPLE 22

### Acide 4-[7-(1-adamantyl)-6-éthoxycarbonylbutyloxy-2-naphyl]benzoïque.

### (a) 4-[7-(1-adamantyl)-6-éthoxycarbonylbutyloxy-2-naphtyl]benzoate d'allyle.

De manière analogue à l'exemple 12(a) par réaction de 2 g (4,5 mmoles) de 4-[7-(1-adamantyl)-6-hydroxy-2-naphtyl]benzoate d'allyle avec 1,4 g (6,7 mmoles) de 5-bromovalérate d'éthyle on obtient 1,55 g (59%) de produit attendu de point de fusion 117-8°C.

### (b) acide 4-[7-(1-adamantyl)-6-éthoxycarbonylbutyloxy-2-naphtyl]benzoïque.

De manière analogue à l'exemple 21(b) à partir 1,48 g (2,6 mmoles) de l'ester précédent, on obtient 1,19 g (87%) d'acide 4-[7-(1-adamantyl)-6-éthoxycarbonyl butyloxy-2-naphtyl]benzoïque de point de fusion 200°C avec décomposition.

### EXEMPLE 23

### Acide 4-[7-(1-adamantyl)-6-carboxypentyloxy-2-naphtyl]benzoïque.

De manière analogue à l'exemple 1(d) à partir de 690 mg (1,28 mmole) d' acide 4-[7-(1-adamantyl)-6-éthoxycarbonylpentyloxy-2-naphtyl]benzoïque, on obtient 250 mg (39%) de l'acide attendu de point de fusion 305-6°C.

### EXEMPLE 24

### Acide 4-[7-(1-admantyl)-6-carboxybutyloxy-2-naphtyl]benzoïque.

De manière analogue à l'exemple 1(d) à partir de 730 mg (1,4 mmole) d'acide 4-[7-(1-adamantyl)-6-éthoxycarbonylbutyloxy-2-naphtyl]benzoïque, on obtient 510 mg (90%) de l'acide attendu de point de fusion 317-8°C.

### EXEMPLE 25

### 4-[7-(1-adamantyl)-6-méthoxyéthoxyméthoxy-2-naphtyl]benzèneméthanol.

Dans un tricol et sous courant d'azote on introduit 3 g (6,1 mmoles) d' acide 4-[7-(1-adamantyl)-6-méthoxyéthoxyméthoxy-2-naphtyl]benzoïque et 40 ml de THF. On ajoutte goutte à goutte 20 ml d'une solution de borane (1 M dans le THF) et chauffe à 40°C pendant une heure. On verse le milieu réactionnel dans une solution d'acide chlorhydrique 1N, extrait avec de l'acétate d'éthyle, décante la phase organique, sèche sur sulfate de magnésium, évapore. On purifie le résidu obtenu par chromatographie sur colonne de silice éluée avec un mélange d'heptane et d'acétate d'éthyle (60-40). On recueille 2,44 g (84%) de 4-[7-(1-adamantyl)-6-méthoxyéthoxyméthoxy-2-naphtyl]benzèneméthanol de point de fusion 131-2°C.

### EXEMPLE 26

### 4-[7-(1-adamantyl)-6-méthoxyéthoxyméthoxy-2-naphtyl]benzaldéhyde.

Dans un ballon on introduit 2,63 g (5,9 mmoles) de 4-[7-(1-adamantyl)-6-méthoxyéthoxyméthoxy-2-naphtyl]benzèneméthanol et 40 ml de dichlorométhane et ajoute 4,2 g de pyridinium dichromate. On agite à température ambiante pendant douze heures, filtre le milieu réactionnel sur silice et évapore le filtrat. Le solide obtenu est recristallisé dans l'heptane, on recueille 490 mg (19%) de 4-[7-(1-adamantyl)-6-méthoxyéthoxyméthoxy-2-naphtyl]benzaldéhyde de point de fusion 83-4°C.

### EXEMPLE 27

### Morpholide de l'acide 4-[7-(1-adamantyl)-6-méthoxyéthoxyméthoxy-2-naphtyl]benzoïque.

### (a) chlorure de l'acide 4-[7-(1-adamantyl)-6-méthoxyéthoxyméthoxy-2-naphtyl] benzoïque.

Dans un ballon on introduit 10 g (20,6 mmoles) d'acide 4-[7-(1-adamantyl)-6-méthoxyéthoxyméthoxy-2-naphtyl]benzoïque et 100 ml de dichlorométhane et ajoute goutte à goutte 4,3 ml (21,6 mmoles) de dicyclohexylamine. On agite à température ambiante pendant une heure et ajoute goutte à goutte 2,2 ml (21,6 mmoles) de chlorure de thionyle. On agite pendant une heure, évapore à sec, reprend par l'éther éthylique, filtre le sel de dicyclohexylamine, évapore le filtrat. On recueille 10,4 g (100%) de chlorure d'acide brut qui sera utilisé tel quel pour la suite de la synthèse.

### (b) Morpholide de l'acide 4-[7-(1-adamantyl)-6-méthoxyéthoxyméthoxy-2-naphtyl] benzoïque.

Dans un ballon on introduit 1,8 ml (20,6 mmoles) de morpholine et 40 ml de THF. On ajoute goutte à goutte une solution de 3,5 g (6,9 mmoles) de chlorure de l'acide 4-[7-(1-adamantyl)-6-méthoxyéthoxyméthoxy-2-naphtyl]benzoïque dans le THF et agite à température ambiante pendant deux heures. On verse le milieu réactionnel dans l'eau, extrait avec de l'acétate d'éthyle, décante la phase organique, sèche sur sulfate de magnésium, évapore. On triture le solide obtenu dans l'hexane, filtre, sèche. On recueille 2,8 g (74%) de l'amide attendu de point de fusion 86-7°C.

### EXEMPLE 28

### N-éthyl-4-[7-(1-adamantyl)-6-méthoxyéthoxyméthoxy-2-naphtyl]benzamide.

De manière analogue à l'exemple 27(b) par réaction de 3,5 g (6,9 mmoles) de chlorure de l'acide 4-[7-(1-adamantyl)-6-méthoxyéthoxyméthoxy-2-naphtyl] benzoïque avec 1,7 ml (20,6 mmoles) d'éthylamine (70%), on obtient 2,6 g (75%) de l'amide éthylique attendu de point de fusion 154-5°C.

### EXEMPLE 29

### 4-[7-(1-adamantyl)-6-méthoxyéthoxyméthoxy-2-naphtyl]benzamide.

De manière analogue à l'exemple 27(b) par réaction de 3,5 g (6,9 mmoles) de chlorure de l'acide 4-[7-(1-adamantyl)-6-méthoxyéthoxyméthoxy-2-naphtyl] benzoïque avec 1,5 ml (26,2 mmoles) d'ammoniaque (32%), on obtient 2,9 g (89%) de l'amide attendu de point de fusion 198-9°C.

### EXEMPLE 30

### N-(4-hydoxyphényl-4-[7-(1-adamantyl)-6-méthoxythoxyméthoxy-2-naphtyl]benzamide.

### (a) N-(4-acétoxyphényl)4-[7-(1-adamantyl)-6-méthoxyéthoxyméthoxy-2-naphtyl] benzamide.

Dans un ballon on introduit 970 mg (6,4 mmoles) de 4-acétoxyaniline 50 ml de THF et 990 µl (7 mmoles) de triéthylamine. On ajoute goutte à goutte une solution de 3,2 g (6,4 mmoles) de chlorure de l'acide 4-[7-(1-adamantyl)-6-méthoxyéthoxy méthoxy-2-naphtyl]benzoïque dans le THF et agite à température ambiante pendant deux heures. On verse le milieu réactionnel dans l'eau, extrait avec de l'acétate d'éthyle, décante la phase organique, sèche sur sulfate de magnésium, évapore. Le résidu obtenu est trituré dans l'heptane filtré, séché. On recueille 3,15 g (81%) du produit attendu de point de fusion 206-7°C.

### (b) N-(4-hydroxyphényl)4-[7-(1-adamantyl)-6-méthoxyéthoxyméthoxy-2-naphtyl] benzamide.

De manière analogue à l'exemple 1(d) à partir de 3,15 g (5,2 mmoles) de N-(4-acétoxyphényl)4-[7-(1-adamantyl)-6-méthoxyéthoxyméthoxy-2-naphtyl] benzamide, on obtient 2,3 g (77%) de N-(4-hydroxyphényl)-4-[7-(1-adamantyl)-6-méthoxyéthoxyméthoxy-2-naphtyl]benzamide de point de fusion 231-3°C.

### EXEMPLE 31

### 4-[7-(1-adamantyl)-6-méthoxyéthoxyméthoxy-2-naphyl]benzoylpipérazine

### (a) N-benzyl-4-[7-(1-adamantyl)-6-méthoxyéthoxyméthoxy-2-naphtyl]benzoyl pipérazine

De manière analogue à l'exemple 27(b) par réaction de 7,3 g (14,4 mmoles) de chlorure de l'acide 4-[7-(1-adamantyl)-6-méthoxyéthoxyméthoxy-2-naphtyl] benzoïque avec 2,5 ml (14,4 mmoles) de 4-benzylpipérazine, on obtient 3 g (33%) du produit attendu de point de fusion 176-7°C.

### (b) 4-[7-(1-adamantyl)-6-méthoxyéthoxyméthoxy-2-naphtyl]benzoylpipérazine

Dans un tricol on introduit 500 mg (0,8 mmole) de l'amide précédent et 20 ml de méthanol. On ajoute 900 mg de Pd/C (10%) puis 900 µl d'acide formique et agite à température ambiante pendant trois heures. On filtre le catalyseur, verse le filtrat dans l'eau bicarbonatée, extrait avec l'acétate d'éthyle, décante la phase organique, sèche sur sulfate de magnésium, évapore. On purifie le résidu obtenu par chromatographie sur colonne de silice éluée avec un mélange de dichlorométhane et de méthanol (80-20). On recueille 310 mg (72%) du produit attendu de point de fusion 177-8°C.

### EXEMPLE 32

### 4-[7-(1-adamantyl)-6-méthoxyéthoxyméthoxy-2-naphtyl]benzoate de propyle.

Dans un tricol et sous courant d'azote, on introduit 3 g (6,1 mmoles) d'acide 4-[7-(1-adamantyl)-6-méthoxyéthoxyméthoxy-2-naphtyl]benzoïque et 100 ml de DMF. On ajoute par petites quantites 200 mg (6,7 mmoles) d'hydrure de sodium (80% dans l'huile) et agite jusqu'à cessation du dégagement gazeux. On ajoute ensuite 500 µl (6,1 mmoles) d'iodopropane et agite pendant une heure. On verse le milieu réactionnel dans l'eau, extrait avec de l'acétate d'éthyle, décante la phase organique, sèche sur sulfate de magnésium, évapore. Le résidu obtenu est purifié par chromatographie sur colonne de silice éluée avec un mélange d'heptane et d'acétate d'éthyle (80-20). On recueille 1 g (33%) d'ester propylique de point de fusion 121-2°C.

### EXEMPLE 33

### Acétate de 4-[7-(1-adamantyl-6-méthoxéthoxyméthoxy-2-naphtyl]phenyle

De manière analogue à l'exemple 1(c) par réaction de 5 g (12,8 mmoles) d'acide 7-(1-adamantyl)-6-méthoxyéthoxyméthoxy-2-naphtylboronique avec 2,7 g (10,1 mmoles) d'acétate de 4-iodophényle, on obtient 2,88 g (57%) de produit attendu de point de fusion 117-8°C.

### EXEMPLE 34

### 4-[7-(1-adamantyl)-6-méthoxyéthoxyméthoxy-2-naphyl]phenol.

De manière analogue à l'exemple 1(d) à partir de 2,5 g (5 mmoles) d'acétate de 4-[7-(1-adamantyl)-6-méthoxyéthoxyméthoxy-2-naphtyl]phenyle, on obtient 2,2 g (97%) de 4-[7-(1-adamantyl)-6-méthoxyéthoxyméthoxy-2-naphtyl]phenol de point de fusion 126-7°C.

### EXEMPLE 35

### Chlorhydrate de 4-[7-(1-adamantyl)-6-méthoxyéthoxyméthoxy-2-naphtyl]phenoxyéthylmorpholine.

Dans un ballon et sous courant d'azote, on introduit 600 mg (1,32 mmole) de 4-[7-(1-adamantyl)-6-méthoxyéthoxyméthoxy-2-naphtyl]phenol, 550 mg (3,96 mmoles) de carbonate de potassium, 4 mg d'iodure de potassium et 60 ml de méthyléthylcétone. On ajoute 270 mg (1,45 mmole) de chlorhydrate de 4-(2-chloroéthyl)morpholine et chauffe à reflux pendant douze heures. On verse le mileu réactionnel dans l'acide chlorhydrique 1 N, extrait avec de l'éther éthylique, décante la phase organique, sèche sur sulfate de magnésium, évapore.On purifie le résidu obtenu par chromatographie sur colonne de silice éluée avec de l'acétate d'éthyle. Après évaporation des solvants, on recueille une huile. On forme le chlorhydrate par dissolution de l'huile dans l'éther éthylique puis addition d' 1 ml d'une solution méthanolique saturée en HCl. On filtre le sel, le sèche, on recueille 350 mg (44%) du chlorhydrate attendu de point de fusion 123-4°C.

### EXEMPLE 36

### Chlorhydrate de 4-[7-(1-adamantyl)-6-méthoxyéthoxyméthoxy-2-naphtyl]phenoxyéthylpiperidine.

De manière analogue à l'exemple 35 par réaction de 600 mg (1,32 mmole) de 4-[7-(1-adamantyl)-6-méthoxyéthoxyméthoxy-2-naphtyl]phenol avec 310 mg (1,7 mmole) de chlorhydrate de 1-(2-chloroéthyl)pipéridine, on obtient 770 mg (85%) du chlorhydrate attendu de point de fusion 126-7°C.

### EXEMPLE 37

### 4-[7-(1-adamantyl)-6-méthoxyéthoxyméthoxy-2-naphtyl]benzoate d'hexyle.

De manière analogue à l'exemple 32 par réaction de 1,35 g (2,8 mmoles) d'acide 4-[7-(1-adamantyl)-6-méthoxyéthoxyméthoxy-2-naphtyl]benzoïque avec 800 µl (5,6 mmoles) d'iodohexane, on obtient 980 mg (62%) d'ester hexylique de point de fusion 73-4°C.

### EXEMPLE 38

### Acide N-[[4-[7-(1-adamantyl)-6-méthoxyéthoxyméthoxy-2-naphtyl]benzoyl]] glutamique.

### (a) N-[[4-[7-(1-adamantyl)-6-méthoxyéthoxyméthoxy-2-naphtyl]benzoyl]]glutamate de diéthyle.

Dans un ballon on introduit 1,93 g (7,8 mmoles) de chlorhydrate de L-glutamate de diéthyle, 1,13 g (9,24 mmoles) de 4-diméthylaminopyridine et 100 ml de THF. On ajoute goutte à goutte une solution de 3,59 g (7,1 mmoles) de chlorure de l'acide 4-[7-(1-adamantyl)-6-méthoxyéthoxyméthoxy-2-naphtyl]benzoïque dans le THF et agite à température ambiante quatre heures. On verse le milieu réactionnel dans l'eau, extrait avec de l'acétate d'éthyle, décante la phase organique, sèche sur sulfate de magnésium, évapore. On purifie le résidu obtenu par chromatographie sur colonne de silice éluée avec un mélange d'heptane et d'acétate d'éthyle (70-30). On recueille 2,8 g (58%) du produit attendu sous forme d'une huile.

### (b) acide N-[[4-[7-(1-adamantyl)-6-méthoxyéthoxyméthoxy-2-naphtyl]benzoyl]] glutamique

De manière analogue à l'exemple 1(d) à partir de 2,7 g (4,2 mmoles) du diester précédent, on obtient 1,45 g (56%) d'acide N-[[4-[7-(1-adamantyl)-6-méthoxyéthoxyméthoxy-2-naphtyl]benzoyl]] glutamique de point de fusion 137-8°C.

### EXEMPLE 39

### Acide 4-[7-(1-adamantyl)-6-méthoxyhexyloxy-2-naphtyl]benzoïque.

### (a) 4-[7-(1-adamantyl)-6-méthoxyhexyloxy-2-naphtyl]benzoate de méthyle.

De manière analogue à l'exemple 12(a) par réaction de 870 mg (1,7 mmole) de 4-[7-(1-adamantyl)-6-hydroxyhexyloxy-2-naphtyl]benzoate de méthyle avec 160 µl (1,7 mmole) de sulfate de diméthyle, on obtient 100 mg (14%) du produit attendu de point de fusion 141-3°C.

### (b) acide 4-[7-(1-adamantyl)-6-méthoxyhexyloxy-2-naphtyl]benzoïque.

De manière analogue à l'exemple 1(d) à partir de 95 mg (0,18 mmole) de l'ester précédent, on obtient 61 mg (66%) de l'acide attendu de point de fusion 267-9°C.

### EXEMPLE 40

### Acide 4-[7-(1-adamantyl)-6-méthoxymethoxypropyl-2-naphtyl]benzoïque.

### (a) 4-[7-(1-adamantyl)-6-méthoxyméthoxypropyl-2-naphtyl]benzoate de méthyle.

Dans un ballon on introduit 541 mg (1,2 mmole) de 4-[7-(1-adamantyl)-6-hydroxypropyl-2-naphtyl]benzoate de méthyle, 5ml de diméthoxyméthane et ajoute 5 gouttes d'iodure de triméthylsilane. On agite à température ambiante 24 heures, verse le milieu réactionnel dans l'eau, extrait avec de l'éther éthylique, décante la phase organique, sèche sur sulfate de magnésium, évapore. Le résidu obtenu est purifié par chromatographie sur colonne de silice éluée avec un mélange de dichlorométhane et d'heptane (70-30). On recueille 355 mg (59%) de produit attendu de point de fusion 137-8°C.

### (b) acide 4-[7-(1-adamantyl)-6-méthoxyméthoxypropyl-2-naphtyl]benzoïque.

De manière analogue à l'exemple 1(d) à partir de 345 mg (0,69 mmole) de l'ester précédent, on obtient 280 mg (83%) de l'acide attendu de point de fusion 237-8°C.

### EXEMPLE 41

### Acide 4-[7-(1-adamantyl)-6-méthoxyméthoxyethyl-2-naphtyl]benzoïque.

### (a) 4-[7-(1-adamantyl)-6-méthoxyméthoxyéthyl-2-naphtyl]benzoate de méthyle.

De manière analogue à l'exemple 40(a) à partir de 200 mg (0,47 mmole) de 4-[7-(1-adamantyl)-6-hydroxyéthyl-2-naphtyl]benzoate de méthyle, on obtient 156 mg (68%) du produit attendu de point de fusion 145-6°C.

### (b) acide 4-[7-(1-adamantyl)-6-méthoxyméthoxyéthyl-2-naphtyl]benzoïque.

De manière analogue à l'exemple 1(d) à partir de 149 mg (0,3 mmole) de l'ester précédent, on obtient 101 mg (70%) de l'acide attendu de point de fusion 225-7°C.

### EXEMPLE 42

L' activité antagoniste des composés de formule (I) est évaluée dans le test de différenciation des cellules F9 de tératocarcinome embryonnaire de souris (Cancer Research 43, p 5268, 1983).

Ces composés testés à 10⁻⁶ M sont inactifs en tant qu'agonistes dans ce test et inhibent partiellement ou totalement l'effet produit par un rétinoide agoniste sur la morphologie et sur la sécrétion de l'activateur du plasminogène selon le protocole suivant.

Les cellules F9 sont ensemencées dans des clusters 12 puits, les composés sont testés de 10⁻⁹ à 10⁻⁵ M en présence de l'acide all-trans-retinoïque ou d'un rétinoide agoniste synthétique (composé A) : l'acide 4-(5,6,7,8-tétrahydro-5,5,8,8-tétraméthyl-2-naphtylaminométhyl)benzoïque (BASF) à 10⁻⁸ M. Après trois jours d'incubation, on effectue les observations morphologiques et l'on détermine la concentration du composé testé (IC50) qui inhibe de 50% l'effet de l'agoniste sur la sécrétion de l'activateur du plasminogène.

| Ex. No. | Antagoniste contre composé A (10nM) test de différenciation des cellules F9 IC50 (nM) |
|---|---|
| 1 | 5 |
| 3 | 2 |
| 5 | 180 |
| 6 | 500 |
| 14 | 250 |
| 20 | 100 |
| 39 | 450 |

### EXEMPLE 43

Dans cet exemple, on a illustré diverses formulations concrètes à base des composés selon l'invention.

### A- VOIE ORALE

### (a) Comprimé de 0,2 g

| | |
|---|---|
| - Composé préparé à l'exemple 6 | 0,001 g |
| - Amidon | 0,114 g |
| - Phosphate bicalcique | 0,020 g |
| - Silice | 0,020 g |
| - Lactose | 0,030 g |
| - Talc | 0,010 g |
| - Stéarate de magnésium | 0,005 g |

### (b) Suspension buvable en ampoules de 5 ml

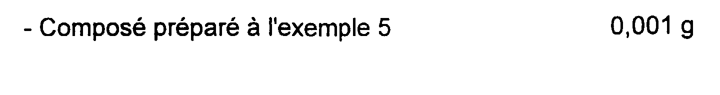

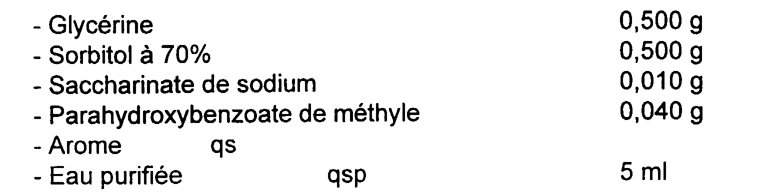

### (c) Comprimé de 0,8 g

| | |
|---|---|
| - Composé de l'exemple 2 | 0,500 g |
| - Amidon prégélatinisé | 0,100 g |
| - Cellulose microcristalline | 0,115 g |
| - Lactose | 0,075 g |
| - Stéarate de magnésium | 0,010 g |

### (d) Suspension buvable en ampoules de 10 ml

| | |
|---|---|
| - Composé de l'exemple 4 | 0,200 g |
| - Glycérine | 1,000 g |
| - Sorbitol à 70% | 1,000 g |
| - Saccharinate de sodium | 0,010 g |
| - Parahydroxybenzoate de méthyle | 0,080 g |
| - Arome qs | |
| - Eau purifiée qsp | 10 ml |

### B- VOIE TOPIQUE

### (a) Onguent

| | |
|---|---|
| - Composé de l'exemple 6 | 0,020 g |
| - Myristate d'isopropyle | 81,700 g |
| - Huile de vaseline fluide | 9,100 g |
| - Silice ("Aérosil 200" vendue par DEGUSSA) | 9,180 g |

### (b) Onguent

| | |
|---|---|
| - Composé de l'exemple 2 | 0,300 g |
| - Vaseline blanche codex qsp | 100 g |

### (c) Crème Eau-dans-Huile non ionique

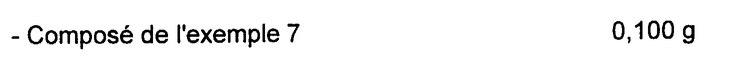

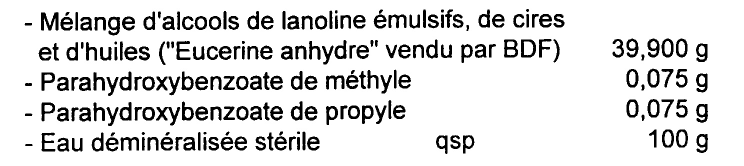

### (d) Lotion

| | |
|---|---|
| - Composé de l'exemple 8 | 0,100 g |
| - Polyéthylène glycol (PEG 400) | 69,900 g |
| - Ethanol à 95% | 30,000 g |

### (e) Onguent hydrophobe

| | |
|---|---|
| - Composé de l'exemple 10 | 0,300 g |
| - Miristate d'isopropyle | 36,400 g |
| - Huile de silicone ("Rhodorsil 47 V 300" vendu par RHONE-POULENC) | 36,400 g |
| - Cire d'abeille | 13,600 g |
| - Huile de silicone ("Abil 300.000 cst" vendu par GOLDSCHMIDT) qsp | 100 g |

### (f) Crème Huile-dans-Eau non ionique

| | |
|---|---|
| - Composé de l'exemple 5 | 1,000 g |
| - Alcool cétylique | 4,000 g |
| - Monostéarate de glycérole | 2,500 g |
| - Stéarate de PEG 50 | 2,500 g |
| - Beurre de karité | 9,200 g |
| - Propylène glycol | 2,000 g |
| - Parahydroxybenzoate de méthyle | 0,075 g |
| - Parahydroxybenzoate de propyle | 0,075 g |
| - Eau déminéralisée stérile qsp | 100 g |

## Revendications

1. Utilisation d'une quantité efficace d'au moins un composé de type rétinoïdes de formule (I) en tant qu'agent actif dans une composition cosmétique ou pour la fabrication d'une composition pharmaceutique, la composition cosmétique ou la composition pharmaceutique étant destinée au traitement de désordres ou d'affections liés à une surrégulation des récepteurs RARs et/ou à une hypervitaminose A, ce composé présentant une formule générale (I) : 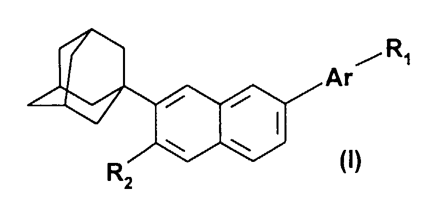 dans laquelle :
- R₁ représente
(i) le radical -CH₃
(ii) le radical -CH₂OH
(iii) le radical -O-R₃
(iv) le radical -CO-R₄
R₃ et R₄ ayant les significations données ci-après,
- Ar est un radical de formule : 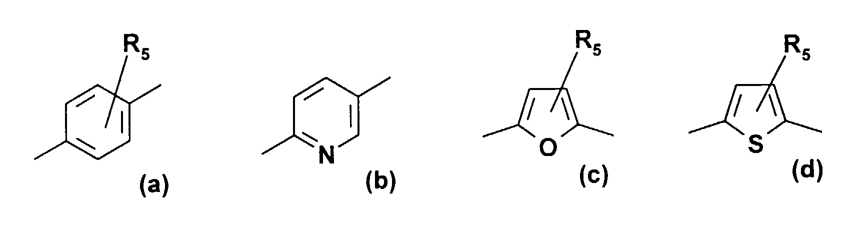 R₅ ayant la signification donnée ci-après,
- R₂ représente:
(a) un radical -(X)ₙ-(CH₂)ₚR₆
(b) un radical -(X)ₙ-(CH₂)q-R₇
(c) un radical -CH=CH-(CH₂)ₛ-R₆
(d) un radical -CH=CH-(CH₂)ₜ-R₇
R₆, R₇, X, n, p, q, s et t ayant les significations données ci-après, étant entendu que :
- R₃ représente un atome d'hydrogène, un radical alkyle inférieur, un radical -(CH₂)ₘ-(CO)ₙ-R₈.
R₈, m et n ayant les significations données ci-après,
- R₄ représente:
(a) un atome d'hydrogène,
(b) un radical alkyle inférieur,
(c) un radical de formule : 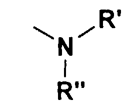
(d) un radical -OR₉,
R', R" et R₉ ayant les significations données ci-après,
- R₅ représente un atome d'hydrogène, d'halogène, un radical alkyle linéaire ou ramifié ayant de 1 à 20 atomes de carbone, un radical hydroxy, un radical -OR₁₀ ou -OCOR₁₀,
R₁₀ ayant la signification donnée ci-après,
- R₆ représente un atome d'hydrogène, un radical alkyle linéaire ou ramifié ayant de 1 à 20 atomes de carbone, un radical alkényle, un radical alkynyle,
- R₇ représente un radical aryle, un radical mono ou polyhydroxyalkyle dont les hydroxy sont éventuellement protégés sous forme de méthoxy ou d'acétoxy ou d'acétonide, un radical amino-alkyle dont la fonction amine est éventuellement substituée par un ou deux groupements alkyles inférieurs, un radical polyéther, un radical -COR₄, un radical hétérocycle saturé ou insaturé ou encore un radical amino-aryle,
- R₈ représente un radical alkyle inférieur ou un hétérocycle saturé,
- R₉ représente un atome d'hydrogène, un radical alkyle linéaire ou ramifié, ayant de 1 à 20 atomes de carbone, un radical alkényle, un radical mono ou polyhydroxyalkyle, un radical aryle ou aralkyle éventuellement substitué(s) ou un reste de sucre ou un reste d'amino acide ou de peptide,
- R₁₀ représente un radical alkyle inférieur,
- X représente un atome d'oxygène ou un radical -S(O)ᵣ,
- R' et R", identiques ou différents, représentent un atome d'hydrogène, un radical alkyle inférieur, un radical mono- ou poly-hydroxyalkyle, un radical aryle éventuellement substitué ou un reste d'amino acide, de peptide ou de sucre ou encore pris ensemble forment un hétérocycle saturé,
- m est un nombre entier compris inclusivement entre 1 et 3,
- n est un nombre entier compris inclusivement entre 0 ou 1,
- p est un nombre entier compris inclusivement entre 5 et 12,
- q est un nombre entier compris inclusivement entre 0 et 12,
- r est un nombre entier compris inclusivement entre 0 et 2,
- s est un nombre entier compris inclusivement entre 3 et 10,
- t est un nombre entier compris inclusivement entre 0 et 10,
ainsi que leurs sels, et leurs analogues chiraux.

2. Utilisation selon la revendication 1, caractérisée en ce que les composés de formule (I) se présentent sous forme de sels d'un métal alcalin ou alcalinoterreux, ou encore de zinc ou d'une amine organique.

3. Utilisation selon l'une des revendications 1 ou 2, caractérisée en ce que les radicaux alkyles inférieurs sont choisis dans le groupe constitué par les radicaux méthyle, éthyle, isopropyle, butyle, tertiobutyle et hexyle.

4. Utilisation selon l'une quelconque des revendications précédentes, caractérisée en ce que les radicaux alkyles linéaires ou ramifiés ayant de 1 à 20 atomes de carbone sont choisis dans le groupe constitué par les radicaux méthyle, éthyle, propyle, 2-éthyl-hexyle, octyle, dodécyle, hexadécyle et octadécyle.

5. Utilisation selon l'une quelconque des revendications précédentes, caractérisée en ce que les radicaux monohydroxyalkyles sont choisis dans le groupe constitué par les radicaux hydroxyméthyle, 2-hydroxyéthyle, 2-hydroxypropyle ou 3-hydroxypropyle.

6. Utilisation selon l'une quelconque des revendications précédentes, caractérisée en ce que les radicaux polyhydroxyalkyles sont choisis dans le groupe constitué par les radicaux 2,3-dihydroxypropyle, 2,3,4-trihydroxybutyle, 2,3,4,5-tétrahydroxypentyle ou le reste du pentaérythritol.

7. Utilisation selon l'une quelconque des revendications précédentes, caractérisée en ce que le radical aryle est un radical phényle éventuellement substitué par au moins un atome d'halogène, un hydroxyle ou une fonction nitro.

8. Utilisation selon l'une quelconque des revendications précédentes, caractérisée en ce que les radicaux aralkyles sont choisis dans le groupe constitué par les radicaux benzyle ou phénéthyle éventuellement substitués par au moins un atome d'halogène, un hydroxyle ou une fonction nitro.

9. Utilisation selon l'une quelconque des revendications précédentes, caractérisée en ce que les radicaux alkényles sont choisis dans le groupe constitué par les radicaux contenant de 2 à 5 atomes de carbone et présentant une ou plusieurs insaturations éthyléniques, en particulier le radical allyle.

10. Utilisation selon l'une quelconque des revendications précédentes, caractérisée en ce que les restes de sucre sont choisis dans le groupe constitué par les restes de glucose, de galactose, de mannose ou d'acide glucuronique.

11. Utilisation selon l'une quelconque des revendications précédentes, caractérisée en ce que les restes d'aminoacide sont choisis dans le groupe constitué par les restes dérivant de la lysine, de la glycine ou de l'acide aspartique.

12. Utilisation selon l'une quelconque des revendications précédentes, caractérisée en ce que les restes de peptide sont choisis dans le groupe constitué par les restes de dipeptide ou de tripeptide.

13. Utilisation selon l'une quelconque des revendications précédentes, caractérisée en ce que les radicaux hétérocycliques saturés sont choisis dans le groupe constitué par les radicaux pipéridino, morpholino, pyrrolidino ou pipérazino, éventuellement substitués en position 4 par un radical alkyle en C₁-C₆ ou mono ou polyhydroxyalkyle.

14. Utilisation selon l'une quelconque des revendications précédentes, caractérisée en ce que les hétérocycles insaturés sont choisis parmi un radical pyridine, furanne ou thiophène.

15. Utilisation selon l'une quelconque des revendications précédentes, caractérisée en ce que les atomes d'halogènes sont choisis dans le groupe constitué par le fluor, le chlore et le brome.

16. Utilisation selon la revendication 1, caractérisée par le fait que les composés sont choisis dans le groupe constitué par:
Acide 2-hydroxy4-[7-(1-adamantyl)-6-benzyloxy-2-naphtyl]benzoïque.
Acide 2-hydroxy-4-[7-(1-adamantyl)-6-hexyloxy-2-naphtyl]benzoïque.
Acide 4-[7-(1-adamantyl)-6-méthoxyéthoxyméthoxy-2-naphtyl]benzoïque.
Acide 5-[7-(1-adamantyl)-6-benzyloxy-2-naphtyl]-2-thiophènecarboxylique.
Acide 4-[7-(1-adamantyl)-6-benzyloxy-2-naphtyl]benzoïque.
Acide 4-[7-(1-adamantyl)-6-benzyloxycarbonyl-2-naphtyl]benzoïque.
Acide 2-hydroxy-4-[7-(1-adamantyl)-6-(4-fluorobenzyl)oxy-2-naphtyl] benzoïque.
Acide 6-[7-(1-adamantyl)-6-méthoxyéthoxyméthoxy-2-naphtyl]nicotinique.
Acide 4-[7-(1-adamantyl)-6-heptyloxy-2-naphtyl]benzoïque.
Acide 2-hydroxy-4-[7-(1-adamantyl)-6-méthoxyéthoxyméthoxy-2-naphtyl] benzoïque.
Acide 2-chloro-4-[7-(1-adamantyl)-6-méthoxyéthoxyméthoxy-2-naphtyl]benzoïque.
Acide 4-[7-(1-adamantyl)-6-hydroxyhexyloxy-2-naphtyl]benzoïque.
Acide 4-[7-(1-adamantyl)-6-hydroxypropyl-2-naphtyl]benzoïque.
Acide 4-[7-(1-adamantyl)-6-hydroxyoctyloxy-2-naphtyl]benzoïque.
Acide 4-[7-(1adamantyl)-6-hydroxyéthyl-2-naphtyl]benzoïque.
Acide 4-[7-(1-adamantyl)-6-hydroxyheptyloxy-2-naphtyl]benzoïque.
Acide 4-[7-(1-adamantyl)-6-hydroxypentyloxy-2-naphtyl]benzoïque.
Acide 4-[7-(1-adamantyl)-6-(4-morpholino)éthyloxy-2-naphtyl]benzoïque.
Acide 4-[7-(1-adamantyl)-6-(1-pipéridino)éthyloxy-2-naphtyl]benzoïque.
Acide4-[7-(1-adamantyl)-6-carbamoylpentyloxy-2-naphtyl]benzoïque.
Acide 4-[7-(1-adamantyl)-6-éthoxycarbonylpentyloxy-2-naphtyl]benzoïque
Acide 4-[7-(1-adamantyl)-6-éthoxycarbonylbutyloxy-2-naphtyl]benzoïque.
Acide 4-[7-(1-adamantyl)-6-carboxypentyloxy-2-naphtyl]benzoïque.
Acide 4-[7-(1-adamantyl)-6-carboxybutyloxy-2-naphtyl]benzoïque.
4-[7-(1-adamantyl)-6-méthoxyéthoxyméthoxy-2-naphtyl]benzéneméthanol.
4-[7-(1-adamantyl)-6-méthoxyéthoxyméthoxy-2-naphtyl]benzaldéhyde.
Morpholide de l'acide 4-[7-(1-adamantyl)-6-méthoxyéthoxyméthoxy-2-naphtyl]benzoïque.
N-éthyl-4-[7-(1-adamantyl)-6-méthoxyéthoxyméthoxy-2-naphtyl]benzamide.
4-[7-(1-adamantyl)-6-méthoxyéthoxyméthoxy-2-naphtyl]benzamide.
N-(4-hydroxyphényl)-4-[7-(1-adamantyl)-6-méthoxyéthoxyméthoxy-2-naphtyl]benzamide.
4-[7-(1-adamantyl)-6-méthoxyéthoxyméthoxy-2-naphtyl]benzoylpipérazine
4-[7-(1-adamantyl)-6-méthoxyéthoxyméthoxy-2-naphtyl]benzoate de propyle.
Acétate de 4-[7-(1-adamantyl)-6-méthoxyéthoxyméthoxy-2-naphtyl]phenyle
4-[7-(1-adamantyl)-6-méthoxyéthoxyméthoxy-2-naphtyl]phenol.
Chlorhydrate de 4-[7-(1-adamantyl)-6-méthoxyéthoxyméthoxy-2-naphtyl]phenoxyéthylmorpholine.
Chlorhydrate de 4-[7-(1-adamantyl)-6-méthoxyéthoxyméthoxy-2-naphtyl]phenoxyéthylpiperidine.
4-[7-(1-adamantyl)-6-méthoxyéthoxyméthoxy-2-naphtyl]benzoate d'hexyle.
Acide N-[[4-[7-(1-adamantyl)-6-méthoxyéthoxyméthoxy-2-naphtyl]benzoyl]]glutamique.
Acide 4-[7-(1-adamantyl)-6-méthoxyhexyloxy-2-naphtyl]benzoïque.
Acide 4-[7-(1-adamantyl)-6-méthoxymethoxyprnpyl-2-naphtyl]benzOÏque.
Acide 4-[7-(1-adamantyl)-6-méthoxyméthoxyethyl-2-naphtyl]benzoïque.

17. Utilisation selon la revendication 1, caractérisée en ce que les composés correspondent à la formule (I) dans laquelle :
- R₁ est le radical -CO-R₄,
- R₂ est le radical : -(X)ₙ-(CH₂)ₚ-R₆ ou -(X)ₙ-(CH₂)_{q}-R₇
- Ar représente le radical de formule (a) ou (b).

18. Utilisation selon l'une quelconque des revendications précédentes, caractérisée en ce que les désordres ou affections liés à une surrégulation des récepteurs RARs et/ou à une hypervitaminose A se traduisent par une composante inflammatoire, allergique et/ou immunologique.

19. Utilisation selon l'une quelconque des revendications précédentes, caractérisée en ce que les composés de formule (I) sont combinés avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques.

20. Utilisation selon l'une quelconque des revendications précédentes, caractérisée en ce que la composition cosmétique ou de préférence pharmaceutique est administrée par voie entérale ou parentérale.

21. Utilisation selon l'une quelconque des revendications 1 à 19, caractérisée en ce que la composition cosmétique ou pharmaceutique est administrée par voie topique ou oculaire.

22. Utilisation selon la revendication précédente, caractérisée en ce que les composés de formule (I) sont utilisés à une concentration comprise entre 0,001% et 5% en poids par rapport au poids total de la composition.

23. Composés, caractérisés en ce qu'ils présentent la formule (I), telle que définie dans l'une quelconque des revendications précédentes, dans laquelle R₇ est un radical polyéther présentant un carbone en position α du carbone qui se trouve en position 6 du radical naphtyl.

24. Composés selon la revendication 23, caractérisés en ce qu'ils sont choisis parmi l'acide 4-[7-(1-adamantyl)-6-méthoxymethoxypropyl-2-naphtyl]benzoïque et l'acide 4-[7-(1-adamantyl)-6-méthoxyméthoxyethyl-2-naphtyl]benzoïque.
